(19) **Europäisches Patentamt / European Patent Office / Office européen des brevets**

(11) **EP 4 782 466 A1**

(12) **EUROPEAN PATENT APPLICATION**
published in accordance with Art. 153(4) EPC

(43) Date of publication:
**29.07.2026  Bulletin 2026/31**

(51) International Patent Classification (IPC):
**C07K 19/00** (2006.01)  **C07K 14/165** (2006.01)
**C12N 9/48** (2006.01)

(21) Application number: **24881599.5**

(22) Date of filing: **22.10.2024**

(86) International application number:
**PCT/CN2024/126450**

(87) International publication number:
**WO 2025/087234 (01.05.2025 Gazette 2025/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **25.10.2023  CN 202311398330**

(71) Applicants:
- **Shanxi Jinbo Bio-Pharmaceutical Co., Ltd.**
  **Shanxi 030032 (CN)**
- **Fudan University**
  **Shanghai 200433 (CN)**

(72) Inventors:
- **LU, Lu**
  **Shanghai 200433 (CN)**
- **JIANG, Shibo**
  **Shanghai 200433 (CN)**
- **XING, Lixiao**
  **Shanghai 200433 (CN)**
- **WANG, Xinling**
  **Shanghai 200433 (CN)**
- **XU, Wei**
  **Shanghai 200433 (CN)**
- **WANG, Qian**
  **Shanghai 200433 (CN)**
- **YANG, Xia**
  **Taiyuan, Shanxi 030032 (CN)**
- **HE, Zhenrui**
  **Taiyuan, Shanxi 030032 (CN)**

(74) Representative: **Cabinet Becker et Associés
25, rue Louis le Grand
75002 Paris (FR)**

Remarks:
The complete document including Reference Table(s) and the Sequence Listing(s) can be downloaded from the EPO website

(54) **FUSION PROTEIN FOR INACTIVATING CORONAVIRUS AND USE THEREOF**

(57)    Provided in the present invention are a fusion protein for inactivating a coronavirus and the use thereof in the treatment of a coronavirus infection. The fusion protein comprises an ACE2 peptidase domain and a coronavirus polypeptide therapeutic agent targeting an HR1 domain, which are directly linked or are linked via a linker. The ACE2 peptidase domain comprises an amino acid sequence as shown in SEQ ID NO: 1.

EP 4 782 466 A1

**Description**

This application claims priority to Chinese Patent Application No. 202311398330.6 filed on October 25, 2023, entitled "FUSION PROTEIN FOR INACTIVATING CORONAVIRUS AND USE THEREOF".

**TECHNICAL FIELD**

The disclosure belongs to the field of biomedicine, and specifically relates to proteins for inactivating SARS-CoV-2 and other ACE2-dependent coronaviruses, and their applications.

**BACKGROUND OF THE ART**

The coronavirus pneumonia (COVID-19) epidemic is caused by severe acute respiratory syndrome coronavirus 2 (SARS-CoV-2). This virus uses the same cellular receptor, ACE2, as severe acute respiratory syndrome coronavirus, but has a stronger transmission capability. With the spread of the virus, prolonged infection duration and growing number of infections, the virus has evolved numerous variants from the prototype strain. Following the designation by WHO of B.1.1.7 (first identified in the UK, Alpha), B.1.351 (first identified in South Africa, Beta), P.1 (first identified in Brazil, Gamma), B.1.617 (first identified in India, Delta), and B.1.529 (first identified in South Africa, Omicron) as variants of concern (VOCs), Omicron has further evolved to generate various subtypes, such as BA.1 to BA.5, BF.7, BQ.1, XBB, EG.5, etc. Although the symptoms caused by the current variant strains infecting adults are relatively mild, the stronger transmissibility and immune escape capabilities produced by the evolution of the virus reduce or eliminate the effectiveness of previously marketed vaccines or antibodies, increasing the chance of reinfection, thereby potentially increasing the risk of developing sequelae and posing safety risks to human health. Based on the above situation, there is an urgent need to develop a broad-spectrum, highly effective antiviral agent designed according to the conserved targets of the virus that may eliminate the adverse effects following viral infection.

SARS-CoV-2 infects target cells through the Spike (S) protein, which consists of homotrimers, and each monomer comprises two subunits, S1 and S2. The receptor binding domain (RBD) in the S1 subunit interacts with the cellular receptor ACE2. In addition, host protease cleavage causes a conformational change in the S2 subunit, the fused peptide at the N-terminus is inserted into the target cell membrane, and the N-terminal heptapeptide repeat region (NHR or HR1) trimer is exposed. Subsequently, three molecules of the C-terminal heptapeptide repeat region (CHR or HR2) are inserted into the hydrophobic groove of the HR1 trimer in an antiparallel manner to form a 6-Helix Bundle (6-HB), thereby bringing the viral envelope and the target cell membrane into close proximity and mediating membrane fusion, and the genetic material of the virus enters the target cell through the fusion pore for replication to produce new viral particles.

In the development of broad-spectrum coronavirus entry inhibitors, the helical region in the S2 protein is an important target. S2P6 is an antibody targeting the stem helix region in the SARS-CoV-2 S2 protein. It has broad-spectrum neutralizing activity against MERS-CoV, SARS-CoV, HCoV-OC43, 229E, etc., but its IC50 (half maximal inhibitory concentration) is relatively high and its activity is suboptimal.

Chinese patent application CN107022008A discloses three engineered polypeptides (including EK1 polypeptide) derived from HCoV-OC43 HR2, which are broad-spectrum and highly effective coronavirus entry inhibitors and can bind to the HR1 trimer exposed after the coronavirus is stimulated by the receptor, thereby competitively inhibiting the binding of the virus's own HR2, and subsequently inhibiting the formation of fusion pores and preventing the virus from entering target cells.

Chinese patent application CN115057914A discloses stapled and alanine-modified EK1 polypeptides. In antiviral activity assays, such polypeptide molecules have been demonstrated to possess anti-SARS-CoV-2 activity, showing potential value during the COVID-19 pandemic.

There remains an urgent need in the art to develop a broad-spectrum, highly effective therapeutic agent capable of inactivating free viral particles at an early stage.

**SUMMARY OF THE DISCLOSURE**

The present disclosure is in part based on the inventors' discovery that currently developed polypeptides, protein therapeutics or small-molecule compounds based on viral replication-related enzymes cannot exert a virus-inactivating effect, and it is difficult to exert inhibitory effects when the virus particles are free and are not adsorbed to cells, which may cause failure to block the initial stage of viral infection. HR1 domain-targeting, anti-coronavirus polypeptide therapeutics, such as EK1 polypeptide, can efficiently inhibit coronavirus infection, but they are ineffective against free virions prior to cellular attachment, which may reduce therapeutic efficacy and limit the opportunity for the therapeutic agent to exert its effect. Therefore, there is an urgent need to develop a broad-spectrum, highly effective therapeutic agent that can exert an inactivating effect during the early free stage of the virus. The inventors have found that after the cell membrane surface receptor analog (free ACE2 protein) interacts with the S1 subunit of the viral envelope protein, this interaction can promote conformational changes in the S2 subunit to expose the binding sites for HR1 domain-targeting, anti-coronavirus polypeptide therapeutics, such as the EK1 polypeptide. Therefore, the fusion protein of ACE2 protein and an HR1 domain-targeting, anti-coronavirus polypeptide therapeutic agent, such as the EK1 polypeptide, is feasible as a highly effective therapeutic agent for inactivating viral infections.

The present disclosure has been accomplished mainly on the basis of the following insights.

The present inventors have conducted in-depth research on the subject matter, and found through previous studies that secreted ACE2 present in the human body can act as an analog of cell membrane-bound ACE2, blocking the RBD in the virus's S protein to prevent viral infection. On this basis, it was found for the first time that the peptidase binding domain of secreted ACE2 protein can induce conformational changes in the S protein to promote the binding of HR1-targeting inhibitors such as EK1 polypeptide (which is considered impossible in the traditional understanding of single receptor-mediated viral infection). Based on the above novel discovery that secreted receptors induce the allosterism of viral proteins, the inventors creatively designed a dual-target viral infection inhibitor protein coupled (e.g., via a linker) between the ACE2 peptidase binding domain and an HR1 target inhibitor such as an EK1 polypeptide. Because the conformational change of the S protein induced by the peptidase binding domain of ACE2 alone is unstable, there may be a potential risk of promoting viral infection, while the coupled HR1 target inhibitor such as EK1 polypeptide can bind to the HR1 target exposed after the S protein undergoes allosterism to further stabilize the S protein structure, thus making the virus completely lose the ability to reinfect cells. Therefore, the fusion protein designed by the present disclosure has the effect of directly inactivating free virus particles in addition to the traditional viral infection inhibitory effect.

In an aspect, provided is a fusion protein comprising an ACE2 peptidase domain and an HR1 domain-targeting, anti-coronavirus polypeptide therapeutic agent, which are directly linked or linked via a linker, wherein the ACE2 peptidase domain comprises (1) the amino acid sequence set forth in SEQ ID NO: 1; (2) an amino acid sequence having 80% or more, 85% or more, 90% or more, 95% or more, 98% or more, or 99% or more sequence identity to the amino acid sequence set forth in SEQ ID NO: 1; or (3) an amino acid sequence with one or more amino acid residue mutation(s) in the amino acid sequence set forth in SEQ ID NO: 1.

SEQ ID NO: 1

MSSSSWLLLSLVAVTAAQSTIEEQAKTFLDKFNHEAEDLFYQSSLASWNYNTNITEENVQNMNNAGDKW
SAFLKEQSTLAQMYPLQEIQNLTVKLQLQALQQNGSSVLSEDKSKRLNTILNTMSTIYSTGKVCNPDNPQE
CLLLEPGLNEIMANSLDYNERLWAWESWRSEVGKQLRPLYEEYVVLKNEMARANHYEDYGDYWRGDY
EVNGVDGYDYSRGQLIEDVEHTFEEIKPLYEHLHAYVRAKLMNAYPSYISPIGCLPAHLLGDMWGRFWTN
LYSLTVPFGQKPNIDVTDAMVDQAWDAQRIFKEAEKFFVSVGLPNMTQGFWENSMLTDPGNVQKAVCH
PTAWDLGKGDFRILMCTKVTMDDFLTAHHEMGHIQYDMAYAAQPFLLRNGANEGFHEAVGEIMSLSAA
TPKHLKSIGLLSPDFQEDNETEINFLLKQALTIVGTLPFTYMLEKWRWMVFKGEIPKDQWMKKWWEMKR
EIVGVVEPVPHDETYCDPASLFHVSNDYSFIRYYTRTLYQFQFQEALCQAAKHEGPLHKCDISNSTEAGQK
LFNMLRLGKSEPWTLALENVVGAKNMNVRPLLNYFEPLFTWLKDQNKNSFVGWSTDWSPYAD

In an embodiment, the anti-coronavirus polypeptide therapeutic agent comprises a polypeptide or derivative thereof selected from the group consisting of: SEQ ID NO: 2 (SLDQINVTFLDLEYEMKKLEEAIKKLEESYIDLKEL), SEQ ID NO: 12 (SLDQINVTFLDLEYEMKKLEEAIKKLEESYIDLKELGSGGRRRRRR), and SEQ ID NO: 6 (NPGDINVTFLDLEYEMKKLEEAIKKLEESYIDLKKL) or an amino acid sequence having 80% or more, 85% or more, 90% or more, 95% or more, 98% or more, or 99% or more sequence identity to any one of SEQ ID NOs: 2, 12, and 6.

In an embodiment, the derivative is a polyethylene glycol-modified, cholesterol-modified derivative of the polypeptide.

In an embodiment, the mutation is selected from a substitution, an addition, an insertion or a deletion.

In an embodiment, the substitution is a conservative amino acid substitution.

In an embodiment, the linker is a flexible linker, preferably $(G)_{n1}$, $(SGG)_{n2}$, $(SGGG)_{n3}$, $(GGSGG)_{n4}$, $(GSSGG)_{n5}$ or $(GGGGS)_{n6}$, wherein n1 = 5-35, 10-30 or 20-25; n2 = 1-13, 2-10 or 3-8; n4, n5 or n6 = 1-10, 2-7 or 3-6, for example n4, n5 or n6 = 5. In an embodiment, the linker is $(GGGGS)_{n6}$, for example $n_6$ is 1, 2, 3, 4, 5, 6 or 7.

In an embodiment, the ACE2 peptidase domain is at the N-terminus of the fusion protein and the anti-coronavirus polypeptide therapeutic agent is at the C-terminus of the fusion protein.

In an embodiment, the fusion protein comprises a half-life-extending moiety, such as an IgG Fc-binding peptide, such as the amino acid sequence of SEQ ID NO: 13 (DCAWHLGELVWCT) or a variant thereof with a substitution, insertion, deletion, or addition(s) of one or more amino acids.

In an embodiment, the fusion protein comprises ACE2 peptidase domain-first linker-EK1 polypeptide or EK1 polypeptide-first linker-ACE2 peptidase domain, and the IgG Fc-binding peptide is linked directly or via a third linker to the ACE2 peptidase domain or the EK1 polypeptide.

In an embodiment, the third linker is $(G)_{m1}$, $(SGG)_{m2}$, $(SGGG)_{m3}$, $(GGSGG)_{m4}$, $(GSSGG)_{m5}$ or $(GGGGS)_{m6}$,

wherein m1 = 5-35, 10-30 or 20-25; m2 = 1-13, 2-10 or 3-8; m4, m5 or m6 = 1-10, 2-7 or 3-6, for example m4, m5 or m6 = 5.

In an embodiment, the fusion protein comprises the amino acid sequence set forth in SEQ ID NO: 7.

SEQ ID NO: 7:

MSSSSWLLLLSLVAVTAAQSTIEEQAKTFLDKFNHEAEDLFYQSSLASWNYNTNITEENVQNMNNAGDKW
SAFLKEQSTLAQMYPLQEIQNLTVKLQLQALQQNGSSVLSEDKSKRLNTILNTMSTIYSTGKVCNPDNPQE
CLLLEPGLNEIMANSLDYNERLWAWESWRSEVGKQLRPLYEEYVVLKNEMARANHYEDYGDYWRGDY
EVNGVDGYDYSRGQLIEDVEHTFEEIKPLYEHLHAYVRAKLMNAYPSYISPIGCLPAHLLGDMWGRFWTN
LYSLTVPFGQKPNIDVTDAMVDQAWDAQRIFKEAEKFFVSVGLPNMTQGFWENSMLTDPGNVQKAVCH
PTAWDLGKGDFRILMCTKVTMDDFLTAHHEMGHIQYDMAYAAQPFLLRNGANEGFHEAVGEIMSLSAA
TPKHLKSIGLLSPDFQEDNETEINFLLKQALTIVGTLPFTYMLEKWRWMVFKGEIPKDQWMKKWWEMKR
EIVGVVEPVPHDETYCDPASLFHVSNDYSFIRYYTRTLYQFQFQEALCQAAKHEGPLHKCDISNSTEAGQK
LFNMLRLGKSEPWTLALENVVGAKNMNVRPLLNYFEPLFTWLKDQNKNSFVGWSTDWSPYADGGGGSG

GGGSGGGGSGGGGSGGGGSSLDQINVTFLDLEYEMKKLEEAIKKLEESYIDLKEL.

In an embodiment, the C-terminus of the fusion protein further comprises an enzyme cleavage site and/or a purification tag.

In an embodiment, the enzyme cleavage site is an HRV 3C enzyme cleavage site.

In an embodiment, the purification tag is a His tag, such as an 8xHis tag.

In an embodiment, the fusion protein comprises the amino acid sequence set forth in SEQ ID NO: 5 or SEQ ID NO: 7.

In another aspect, provided is a nucleic acid encoding the fusion protein according to the present disclosure.

In an embodiment, the nucleic acid comprises a codon-optimized nucleotide sequence.

In an embodiment, the nucleotide sequence is a nucleotide sequence that is codon-optimized for expression in a eukaryotic cell or a prokaryotic cell, such as yeast or *E. coli.*

In another aspect, provided is a vector comprising the nucleic acid described herein.

In an embodiment, the vector comprises an expression control element, a nucleotide of a purification tag and/or a nucleotide of a leader sequence operably linked to the nucleic acid.

In an embodiment, the expression control element is selected from a promoter, a terminator, or an enhancer.

In an embodiment, the purification tag is selected from a His tag, a GST tag, an MBP tag, a SUMO tag, or a NusA tag.

In an embodiment, the vector is an expression vector, a cloning vector, a shuttle vector, or a viral vector, such as a plasmid or cosmid. For example, the vector is a pFuse-hIgG1-Fc2 series of plasmids or a pET series of plasmids.

In another aspect, provided is a host cell comprising the nucleic acid described herein or the vector according to the disclosure. Preferably, the host cell is a eukaryotic cell or a prokaryotic cell. Preferably, the eukaryotic cell is a yeast cell, an animal cell, and/or an insect cell, and/or the prokaryotic cell is an *E. coli* cell, for example an *E. coli* BL21 cell.

In another aspect, provided is a composition comprising one or more of the fusion protein, nucleic acid, vector, and host cell described herein. Preferably, the composition is a pharmaceutical composition or kit. Preferably, the composition comprises a pharmaceutically acceptable carrier, excipient or diluent. Preferably, the composition is a solid, liquid or gel composition. Preferably, the composition is an injectable composition or an orally administered composition. Preferably, the composition is in the form of a tablet, capsule, drop pill, aerosol, pill, powder, solution, suspension, emulsion, granule, liposome, transdermal agent, suppository, or lyophilized powder. Preferably, the composition further comprises another anti-coronavirus therapeutic agent. Preferably, the therapeutic agent is selected from Paxlovid, amubarvimab / romlusevimab, COVID-19 human immunoglobulin, convalescent plasma, glucocorticoids, and interleukin-6 inhibitors.

In another aspect, provided is a method for producing the fusion protein described herein, comprising

(1) culturing the host cell according to the disclosure under a suitable culture condition;
(2) harvesting the host cell and/or culture medium comprising the fusion protein; and
(3) purifying the fusion protein.

Preferably, step (3) comprises, when the fusion protein comprises a purification tag, isolating the fusion protein by column separation using a ligand for the purification tag and/or removing the purification tag by enzymatic digestion using a tool enzyme. Preferably, the purification tag is a histidine tag, such as an 8xHis tag. Preferably, the column separation is a nickel column separation. Preferably, when the fusion protein comprises an HRV 3C enzyme cleavage site, the tool enzyme is an HRV 3C enzyme. Preferably, the method further comprises the step of modifying the fusion protein.

Preferably, when the ACE2 peptidase domain is located at the N-terminus of the fusion protein and the anti-coronavirus polypeptide therapeutic agent is at the C-terminus of the fusion protein, the method comprises PEGylating or adding a cholesterol moiety to the C-terminus of the anti-coronavirus polypeptide therapeutic agent.

In another aspect, provided is the use of the fusion protein, nucleic acid, vector, host cell, and/or composition according to the disclosure in the manufacture of a medicament or kit for treating or preventing a coronavirus infection or a coronavirus infection-related disease in a subject. Provided is a method for treating or preventing a coronavirus infection or a coronavirus infection-related disease in a subject, comprising administering the fusion protein, nucleic acid, and/or composition according to the disclosure. Preferably, the coronavirus uses ACE2 as a cellular receptor. Preferably, the coronavirus is one or more of severe acute respiratory syndrome coronavirus (SARS-CoV), Middle East respiratory syndrome coronavirus and severe acute respiratory syndrome coronavirus 2 (SARS-CoV-2), HCoV, RsSHC014-CoV, or RsW1V1-CoV. Preferably, the HCoV is selected from HCoV-229E, HCoV-NL63, HCoV-OC43, and HCoV-HKU1 strains. Preferably, the SARS-CoV-2 is selected from the prototype, Alpha, Beta, Gamma, Delta, Lambda, BA.1, BA.2, BA.2.2, BA.2.9, BA.2.12.1, BA.2.75, BA.3, BA.4.6, BA.5, BF.7, XBB, or BQ.1 virus strain. Preferably, the medicament is in the form of a tablet, capsule, drop pill, aerosol, pill, powder, solution, suspension, emulsion, granule, liposome, transdermal agent, suppository, or lyophilized powder. Preferably, the subject is a human or mammal, such as a companion animal, a zoo animal, or a livestock animal. In an embodiment, the coronavirus infection-related disease is coronavirus pneumonia.

In another aspect, provided is a method for non-therapeutical purpose or a method for therapeutical purpose of treating a coronavirus infection, comprising contacting a coronavirus-infected cell with the fusion protein and/or composition described herein; or contacting an uninfected cell with the fusion protein described herein and/or the composition described herein to avoid a coronavirus infection. Preferably, the coronavirus uses ACE2 as a cellular receptor. Preferably, the coronavirus is one or more of severe acute respiratory syndrome coronavirus (SARS-CoV), Middle East respiratory syndrome coronavirus and severe acute respiratory syndrome coronavirus 2 (SARS-CoV-2), HCoV, RsSHC014-CoV or RsW1V1-CoV. Preferably, the HCoV is selected from HCoV-229E, HCoV-NL63, HCoV-OC43 and HCoV-HKU1 strains. Preferably, the SARS-CoV-2 is selected from the prototype, Alpha, Beta, Gamma, Delta, Lambda, BA.1, BA.2, BA.2.2, BA.2.9, BA.2.12.1, BA.2.75, BA.3, BA.4.6, BA.5, BF.7, XBB or BQ.1 virus strain.

In another aspect, provided is a method of inhibiting coronavirus reproduction *in vitro,* or preventing or inhibiting infection of cells by a coronavirus *in vitro,* comprising the step of contacting the fusion protein and/or composition described herein with an article or sample containing or at risk of containing the coronavirus. A method of preventing and/or treating coronavirus infection is provided. Preferably, the coronavirus uses ACE2 as a cellular receptor. Preferably, the coronavirus is one or more of severe acute respiratory syndrome coronavirus (SARS-CoV), Middle East respiratory syndrome coronavirus and severe acute respiratory syndrome coronavirus 2 (SARS-CoV-2), HCoV, RsSHC014-CoV or RsW1V1-CoV. Preferably, the HCoV is selected from HCoV-229E, HCoV-NL63, HCoV-OC43 and HCoV-HKU1 strains. Preferably, the SARS-CoV-2 is selected from the prototype, Alpha, Beta, Gamma, Delta, Lambda, BA.1, BA.2, BA.2.2, BA.2.9, BA.2.12.1, BA.2.75, BA.3, BA.4.6, BA.5, BF.7, XBB or BQ.1 virus strain.

Advantages of the disclosure include:

1. The fusion protein of the present disclosure is capable of binding, through the ACE2 functional domain, to the RBD in the S1 subunit of the spike protein of SARS-CoV-2 and other ACE2-dependent coronaviruses, thereby inducing conformational changes in the S2 subunit. Subsequently, through the EK1 polypeptide, it binds to the exposed HR1 target in the S2 subunit, thereby rendering the free virus particles non-infectious and terminating the viral infection process at the initial stage. Compared to other viral protease inhibitors and entry inhibitors, this significantly improves therapeutic efficacy.

2. By employing a protein-peptide coupling strategy, the inhibitory activity of the ACE2 protein is enhanced by 26- to 600-fold, thereby significantly reducing costs in downstream therapeutic development. Furthermore, this strategy confers viral inactivation capabilities upon the EK1 polypeptide, which originally possessed solely viral infection inhibitory activity.

3. *In vivo* studies in mice demonstrate that the protein disclosed herein, acting as an inhibitor or inactivator, exhibits an improved safety profile and greater potential for therapeutic development.

4. The inventors found that the length of the linker in the fusion protein affects its inhibitory or inactivating properties against coronaviruses, with the AL5E protein exhibiting an optimal inhibitory or inactivating activity.

**DESCRIPTION OF THE DRAWINGS**

FIG. 1 shows the identification results of AL2E, AL5E, AL6E and AL7E proteins by gel filtration chromatography and Coomassie brilliant blue staining.

FIG. 2 shows the screening results for the inhibition of SARS-CoV-2 VOCs and Omicron variant pseudoviruses by AL2E, AL5E, AL6E, and AL7E proteins.

FIG. 3 shows the inhibitory activity of the AL5E protein against the live Omicron variant strain BA.2.2.

FIG. 4 shows the inactivation of SARS-CoV-2 VOCs and Omicron variant pseudoviruses by the AL5E protein.

FIG. 5 shows the inhibition and inactivation efficacy of the AL5E protein against other ACE2-dependent coronaviruses.

FIG. 6 shows the protective efficacy of the AL5E protein against human coronavirus NL63 infection in K18-ACE2 transgenic mice.

FIG. 7 shows the protective efficacy of K18-ACE2 transgenic mice from infection following the inactivation of human coronavirus NL63 by the AL5E protein.

## DETAILED DESCRIPTION OF THE DISCLOSURE

Unless otherwise defined, all technical and scientific terms used herein have the same meaning as commonly understood by one of ordinary skill in the art. Unless otherwise specified, the units used in the present specification are international standard units, and the numerical values and numerical ranges appearing in the present disclosure should be understood to include unavoidable systematic errors. The following describes embodiments of the present disclosure; however, the present disclosure is not limited thereto.

In the present disclosure, the terms "comprising", "having", "including" or "containing" may refer to inclusive or open-ended, and do not exclude additional, unrecited elements or method steps. Furthermore, the terms "comprising", "having", "including" or "containing" may also cover closed-ended expressions excluding additional, unrecited elements or method steps.

In the present disclosure, the term "fusion protein" refers to a novel protein produced by fusing a certain functional protein molecule having a biological activity with other natural protein (fusion partner) using a technique such as genetic engineering. The fusion protein may have two or more proteins with different functions. Furthermore, in order to facilitate processing such as purification, the fusion protein may also further comprise a protein tag and/or a protease cleavage site. The selection of protein tags and protease cleavage sites is known to those skilled in the art and is not particularly limited.

In the present disclosure, the term "amino acid" may include natural amino acids, unnatural amino acids, amino acid analogs, and all of their D and L stereoisomers.

In the present disclosure, an amino acid deletion may refer to the deletion of 1, 2 or 3 or more amino acids from an amino acid sequence, as long as the altered sequence completely or partially retains the activity of the original amino acid sequence.

In the present disclosure, an amino acid addition may refer to the addition of 1, 2, or 3 or more amino acids at the C-terminus, N-terminus, or at any position between the C-terminus and N-terminus of an amino acid sequence, as long as the altered sequence completely or partially retains the activity of the original amino acid sequence.

In the present disclosure, an amino acid substitution refers to the replacement of an amino acid at a specific position in an amino acid sequence with another amino acid, as long as the altered sequence completely or partially retains the activity of the original amino acid sequence. Such a substitution may be a conservative amino acid substitution, which refers to the replacement of several amino acids with amino acids having similar or closely related properties. For example, a conservative substitution may be produced as follows: substituting Val, Leu, or Ile for Ala; substituting Lys, Gln, Asn, or His for Arg; substituting Gln, His, Lys, or Arg for Asn; substituting Glu or Asn for Asp; substituting Ser or Ala for Cys; substituting Asn or Gln for Glu; substituting Ala for Gly; substituting Asn, Lys, Gln, or Arg for His; substituting Leu, Met, Ala, Val, or Phe for Ile; substituting Ile, Met, Ala, Val, or Phe for Leu; substituting Asn, Gln, or Arg for Lys; substituting Ile, Leu, or Phe for Met; substituting Leu, Val, Ile, Ala, or Tyr for Phe; substituting Ala for Pro; substituting Thr for Ser; substituting Ser or Val for Thr; substituting Phe or Tyr for Trp; substituting Trp, Phe, Thr, or Ser for Tyr; and substituting Phe, Ala, Met, Ile, or Leu for Val. Amino acid substitutions may also be non-conservative amino acid substitutions.

In the present disclosure, a "flexible linker" includes, but is not limited to, hydrocarbon linkers and peptide linkers. The peptide linker consists of linear or branched amino acids linked by a peptide bond, for example, a peptide linker comprising small non-polar (e.g., Gly) and/or polar (e.g., Ser or Thr) amino acid residues, specifically Gly, Gly-Gly, Ser-Gly-Gly, Ser-Gly-Gly-Gly, Gly-Gly-Ser-Gly-Gly, Gly-Ser-Ser-Gly-Gly, GSG, and the like. Each linker described herein (e.g., the first, second, or third linker) may be independently selected from any one of the linker sequences, or the linkers having a plurality of repeats of any one of the sequences (e.g., 1, 2, 3, 4, 5, 6, 7, 8, 9, 10, 11, or 12).

The term "one or more" is to be understood to include, but is not limited to, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150, 200, 300, 400, 500, 600, 700, 800, 900, 1000, 2000, 3000, 4000, 5000 or more. Also included are numerical ranges therebetween, such as 1-5000 or 1-10.

As used herein, the term "nucleic acid" means a single-stranded or double-stranded nucleic acid molecule that is isolated from a naturally occurring gene, modified to contain a segment of nucleic acid in a manner not originally existing in nature, or is synthetic, wherein the nucleic acid molecule may comprise one or more control sequences. The nucleic acid may be a codon-optimized nucleic acid, for example, a nucleic acid that is codon-optimized for expression in *E. coli* cells.

The term "operably linked" means a configuration in which a control sequence is placed at an appropriate position relative to a coding sequence of a polynucleotide such that the control sequence directs expression of the coding sequence.

As used herein, "DPEG" refers to discrete polyethylene glycol. DPEG4 indicates that the number of repeating ethylene glycol residues is 4.

The degree of association between two amino acid sequences or between two nucleotide sequences is described by the parameter "sequence identity". For the purposes of the present disclosure, the sequence identity between two amino acid sequences is determined using the Needleman-Wunsch algorithm (Needleman and Wunsch, 1970, J. Mol. Biol. 48: 443-453) as implemented by the Needle program of the EMBOSS software package (EMBOSS: The European Molecular Biology Open Software Suite, Rice et al., 2000, Trends Genet. 16: 276-277), preferably version 5.0.0 or later. The parameters used are a gap open penalty of 10, a gap extension penalty of 0.5, and an EBLOSUM62 (EMBOSS version of BLOSUM62) substitution matrix. The output of the Needle labeled "longest identity" (obtained using the non-simplified option) is used as the percent identity and calculated as follows:

$$(\text{Identical residues} \times 100) / (\text{Length of alignment - Total number of gaps in alignment})$$

For the purposes of the present disclosure, the sequence identity between two deoxynucleotide sequences is determined using the Needleman-Wunsch algorithm (Needleman and Wunsch, 1970, *supra*) as implemented by the Needle program of the EMBOSS software package (EMBOSS: The European Molecular Biology Open Software Suite, Rice et al., 2000, *supra*), preferably version 5.0.0 or later. The parameters used are a gap open penalty of 10, a gap extension penalty of 0.5, and an EDNAFULL (EMBOSS version of NCBI NUC4.4) substitution matrix. The output of the Needle labeled "longest identity" (obtained using the non-simplified option) is used as the percent identity and calculated as follows:

$$(\text{Identical deoxynucleotides} \times 100) / (\text{Length of alignment - Total number of gaps in alignment})$$

In the present disclosure, suitable vectors are known in the art of vector construction, including the selection of promoters and other regulatory elements, such as enhancer elements. The vector described herein includes sequences suitable for introduction into cells. For example, the vector may be an expression vector in which the coding sequence of the fusion protein is controlled by its own cis-acting regulatory elements. The design of the vector facilitates gene integration or gene replacement in the host cell, etc. One of ordinary skill in the art will understand that, in the present disclosure, the term "vector" includes a DNA molecule, for example, a plasmid, a bacteriophage, a virus, or other vector. It may contain one or more heterologous or recombinant nucleotide sequences. Suitable phage and viral vectors include, but are not limited to, λ-phage, EMBL phage, simian virus, bovine papillomavirus, Epstein-Barr virus, adenovirus, herpes virus, mouse sarcoma virus, murine mammary tumor virus, lentivirus, and the like.

In the present disclosure, the host cell may be a eukaryotic cell, such as fungi and yeast, or a prokaryotic cell, such as a bacterium of the family Enterobacteriaceae.

In the present disclosure, any pharmaceutically acceptable excipient includes, but is not limited to, diluents, osmotic pressure regulators, stabilizers, antibacterial agents, disintegrants, binders, fillers, lubricants, antioxidants, flavoring agents, fragrances, co-solvents, emulsifiers, solubilizers, colorants, and the like.

It should be understood that, in practical applications, the proteins of the present disclosure or pharmaceutically acceptable salts thereof, derivatives thereof or pharmaceutically acceptable salts of the derivatives, the conjugates described above, the multimers described above, and the compositions described above may be administered to a patient as medicaments directly or in mixture with suitable carriers or excipients.

In the present disclosure, the administration form of the provided pharmaceutical composition is not limited in any way, and includes, for example, injection (intra-arterial, intravenous, intramuscular, intraperitoneal, subcutaneous), mucosal, oral (oral solid preparation, oral liquid preparation), rectal, inhalation, implantation, topical (e.g., ocular) administration, and the like. Non-limiting examples of oral solid preparations include, but are not limited to, powders, capsules, lozenges, granules, tablets, and the like. Non-limiting examples of liquid preparations for oral or mucosal administration include, but are not limited to, suspensions, tinctures, elixirs, solutions, and the like. Non-limiting examples of topically administered preparations include, but are not limited to, emulsions, gels, ointments, creams, patches, pastes, foams, lotions, drops, or serum preparations. Non-limiting examples of preparations for parenteral administration include, but are not limited to, solutions for injection, dry powders for injection, suspensions for injection, emulsions for injection, and the like. The compositions of the present disclosure may also be formulated into controlled release or delayed release dosage forms (e.g., liposomes or microspheres).

**Problem to be Solved by the Disclosure**

The objective of the present disclosure is to provide a fusion protein which is a protein-type viral infection inactivator having an inactivating effect against SARS-CoV-2 and its variant strains, as well as other ACE2-dependent coronaviruses. The protein-type inactivator can bind to the receptor binding domain (RBD) in the S1 subunit of the viral envelope protein via ACE2, thereby triggering a conformational change of the S2 subunit, exposing the HR1 domain therein, and causing the binding of HR1 domain-targeting, anti-coronavirus polypeptide therapeutic agents, such as the EK1 polypeptide, ultimately rendering the virion non-infectious through a "dual-lock mechanism".

**Solution to the Problem**

The first aspect of the present disclosure provides a fusion protein that inactivates SARS-CoV-2 and its variant strains, as well as other ACE2-dependent coronaviruses. Specifically, in the present disclosure, the ACE2 peptidase domain is linked to an HR1 domain-targeting, anti-coronavirus polypeptide therapeutic agent, such as the EK1 polypeptide, directly or through a flexible linker, to produce a novel fusion protein with dual functional domains.

The ACE2 peptidase domain may be at least 615 contiguous amino acids at the N-terminus of the human ACE2 protein. The HR1 domain-targeting, anti-coronavirus polypeptide therapeutic agent may be an anti-coronavirus polypeptide therapeutic agent such as the EK1 polypeptide, which was disclosed in a patent application previously filed by the present inventors (Chinese Patent Publication No. CN107022008A, entitled "Polypeptide Broadly Inhibiting Human Coronavirus Infection and Use Thereof"). The flexible linker may be composed of 5 short peptides, each consisting of 5 amino acid residues. The C-terminus of the fusion protein may optionally comprise a protein tag, which is linked to the last amino acid at the C-terminus of the protein via an enzymatic cleavage site, such as the HRV 3C enzymatic cleavage site amino acid sequence. Preferably, a fusion protein of ACE2-linker-EK1 (i.e., AL2E, AL5E, AL6E, and AL7E) may be obtained. Particularly preferred is a fusion protein having the amino acid sequence set forth in SEQ ID NO: 5, designated as AL5E. AL2E, AL5E, AL6E, and AL7E differ from one another solely in the linker length, representing (**GGGGS**)$_2$, (**GGGGS**)$_5$, (**GGGGS**)$_6$, and (**GGGGS**)$_7$, respectively.

MSSSSWLLLLSLVAVTAAQSTIEEQAKTFLDKFNHEAEDLFYQSSLASWNYNTNITEENV
QNMNNAGDKWSAFLKEQSTLAQMYPLQEIQNLTVKLQLQALQQNGSSVLSEDKSKRLNTI
LNTMSTIYSTGKVCNPDNPQECLLLEPGLNEIMANSLDYNERLWAWESWRSEVGKQLRPLY
EEYVVLKNEMARANHYEDYGDYWRGDYEVNGVDGYDYSRGQLIEDVEHTFEEIKPLYEHL
HAYVRAKLMNAYPSYISPIGCLPAHLLGDMWGRFWTNLYSLTVPFGQKPNIDVTDAMVDQ
AWDAQRIFKEAEKFFVSVGLPNMTQGFWENSMLTDPGNVQKAVCHPTAWDLGKGDFRIL
MCTKVTMDDFLTAHHEMGHIQYDMAYAAQPFLLRNGANEGFHEAVGEIMSLSAATPKHL
KSIGLLSPDFQEDNETEINFLLKQALTIVGTLPFTYMLEKWRWMVFKGEIPKDQWMKKWW
EMKREIVGVVEPVPHDETYCDPASLFHVSNDYSFIRYYTRTLYQFQFQEALCQAAKHEGPL
HKCDISNSTEAGQKLFNMLRLGKSEPWTLALENVVGAKNMNVRPLLNYFEPLFTWLKDQN
KNSFVGWSTDWSPYADGGGGSGGGGSGGGGSGGGGSGGGGSSLDQINVTFLDLEYEMK
KLEEAIKKLEESYIDLKELLEVLFQGPHHHHHHHH(SEQ ID NO: 5).

The examples of the disclosure do not involve excision of the His tag; however, it will be understood by those skilled in the art that the His tag and the enzymatic cleavage site have no inhibitory effect on the virus. When the His tag and the enzymatic cleavage site are removed, the resulting fusion protein (SEQ ID NO: 7) exhibits the same virus inhibitory effect as the fusion protein of SEQ ID NO: 5.

In the above sequence, the single-underlined portion represents the ACE2 peptidase domain in the AL5E protein, which consists of 615 contiguous amino acids at the N-terminus of the ACE2 protein, the specific sequence of which is shown in SEQ ID NO: 1. The wavy-underlined portion of the above sequence represents the EK1 polypeptide, the specific sequence of which is shown in SEQ ID NO: 2. The bold portion of the above sequence represents the flexible linker in the AL5E protein.

The present disclosure further provides that, in addition to the ACE2 peptidase domain and the EK1 polypeptide, the fusion protein may also comprise a protein or other functional moiety for extending half-life. The structure of such a fusion protein may be represented as: ACE2 peptidase domain - first linker - EK1 polypeptide - second linker - half-life-extending moiety. Fusion proteins comprising such trifunctional properties can be routinely prepared by those skilled in the art. For example, Chinese Patent Application No. 202310423264.7, previously filed by the inventors, describes a half-life-extending moiety, disclosing that a portion of the amino acid sequence of SEQ ID NO: 12 (DCAWHLGELVWCT) can extend the half-life. Furthermore, Chinese Patent Application No. CN202180001804.1, previously filed by the inventors, describes derivatives of anti-coronavirus polypeptide therapeutic agents, wherein it is verified that EK1- palmitate (EK1-palmitic): SLDQINVTFLDLEYEMKKLEEAIKKLEESYIDLKEL-GSG-DPEG4-K(palmitic) and EK1-cholesterol (EK1-chol): SLDQINVTFLDLEYEMKKLEEAIKKLEESYIDLKEL-GSGSG-DPEG4-C(cholesterol) possess inhibitory activity against coronavirus pseudoviruses such as 2019-nCoV. The polypeptide derivatives may have the structure SLDQINVTFLDLEYEMKKLEEAIKKLEESYIDLKEL-(GSG)n-DPEG4-K-palmitic acid or a salt or ester thereof, or SLDQINVTFLDLEYEMKKLEEAIKKLEESYIDLKEL-(GSGSG)n-DPEG4-C-cholesterol or a salt or ester thereof, wherein n is 1 or 2. Those skilled in the art may replace the EK1 polypeptides employed in the examples described herein with such derivatives.

A second aspect of the present disclosure provides a nucleic acid encoding the fusion protein of the present disclosure, wherein the nucleotide sequence of the encoding nucleic acid comprises the sequence shown in SEQ ID NO: 3 or SEQ ID NO: 4, or a derivative thereof following artificial design and codon optimization.

SEQ ID NO: 3 (Nucleotide sequence encoding the ACE2 peptidase domain):

ATGTCAAGCTCTTCCTGGCTCCTTCTCAGCCTTGTTGCTGTAACTGCTGCTCAGTCCACCATTGAGGAA
CAGGCCAAGACATTTTTGGACAAGTTTAACCACGAAGCCGAAGACCTGTTCTATCAAAGTTCACTTGC
TTCTTGGAATTATAACACCAATATTACTGAAGAGAATGTCCAAAACATGAATAATGCTGGGGACAAA
TGGTCTGCCTTTTTAAAGGAACAGTCCACACTTGCCCAAATGTATCCACTACAAGAAATTCAGAATCT
CACAGTCAAGCTTCAGCTGCAGGCTCTTCAGCAAAATGGGTCTTCAGTGCTCTCAGAAGACAAGAGC
AAACGGTTGAACACAATTCTAAATACAATGAGCACCATCTACAGTACTGGAAAAGTTTGTAACCCAG
ATAATCCACAAGAATGCTTATTACTTGAACCAGGTTTGAATGAAATAATGGCAAACAGTTTAGACTAC
AATGAGAGGCTCTGGGCTTGGGAAAGCTGGAGATCTGAGGTCGGCAAGCAGCTGAGGCCATTATATG
AAGAGTATGTGGTCTTGAAAAATGAGATGGCAAGAGCAAATCATTATGAGGACTATGGGATTATTG
GAGAGGAGACTATGAAGTAAATGGGGTAGATGGCTATGACTACAGCCGCGGCCAGTTGATTGAAGAT
GTGGAACATACCTTTGAAGAGATTAAACCATTATATGAACATCTTCATGCCTATGTGAGGGCAAAGTT
GATGAATGCCTATCCTTCCTATATCAGTCCAATTGGATGCCTCCCTGCTCATTTGCTTGGTGATATGTG
GGGTAGATTTTGGACAAATCTGTACTCTTTGACAGTTCCCTTTGGACAGAAACCAAACATAGATGTTA
CTGATGCAATGGTGGACCAGGCCTGGGATGCACAGAGAATATTCAAGGAGGCCGAGAAGTTCTTTGT
ATCTGTTGGTCTTCCTAATATGACTCAAGGATTCTGGGAAAATTCCATGCTAACGGACCCAGGAAATG
TTCAGAAAGCAGTCTGCCATCCCACAGCTTGGGACCTGGGGAAGGGCGACTTCAGGATCCTTATGTG
CACAAAGGTGACAATGGACGACTTCCTGACAGCTCATCATGAGATGGGGCATATCCAGTATGATATG
GCATATGCTGCACAACCTTTTCTGCTAAGAAATGGAGCTAATGAAGGATTCCATGAAGCTGTTGGGG
AAATCATGTCACTTTCTGCAGCCACACCTAAGCATTTAAAATCCATTGGTCTTCTGTCACCCGATTTTC
AAGAAGACAATGAAACAGAAATCAACTTCCTGCTGAAGCAGGCCCTGACCATTGTTGGGACTCTGCC
ATTTACTTACATGTTAGAGAAGTGGAGGTGGATGGTCTTTAAAGGGGAAATTCCCAAAGACCAGTGG
ATGAAAAGTGGTGGGAGATGAAGCGAGAGATAGTTGGGGTGGTGGAACCTGTGCCCCATGATGAA
ACATACTGTGACCCCGCATCTCTGTTCCATGTTTCTAATGATTACTCATTCATTCGATATTACACAAGG
ACCCTTTACCAATTCCAGTTTCAAGAAGCACTTTGTCAAGCAGCTAAACATGAAGGCCCTCTGCACAA
ATGTGACATCTCAAACTCTACAGAAGCTGGACAGAAACTGTTCAATATGCTGAGGCTTGGAAAATCA
GAACCCTGGACCCTAGCATTGGAAAATGTTGTAGGAGCAAAGAACATGAATGTAAGGCCACTGCTCA
ACTACTTTGAGCCCTTATTTACCTGGCTGAAAGACCAGAACAAGAATTCTTTTGTGGGATGGAGTACC
GACTGGAGTCCATATGCAGAC

SEQ ID NO: 4 (nucleotide sequence encoding EK1 polypeptide):

AGCCTGGACCAGATCAACGTGACCTTCCTGGACCTGGAGTACGAGATGAAGAAGCT

GGAGGAGGCCATCAAGAAGCTGGAGGAGAGCTACATCGACCTGAAGGAGCTG

A third aspect of the present disclosure provides a recombinant expression vector. The expression vectors selected in the present disclosure can stably reside and autonomously replicate in various hosts of prokaryotic or eukaryotic cells, such as conventional plasmids in the art (e.g., a pFuse-hIgG1-Fc2 series of plasmids), shuttle vector pNV18.1, phage or viral vectors, and the like. The nucleotide sequence of the present disclosure is cloned into the vector through molecular biological operations such as restriction enzyme digestion and ligation, thereby constructing a recombinant expression vector.

A fourth aspect of the present disclosure provides a recombinant host cell, wherein the host cell is selected from a prokaryotic cell, a yeast, or a eukaryotic cell, and may further be selected from *E. coli, Rhodococcus ruber, Bacillus subtilis,* or yeast. The recombinant expression vector can be transformed into the host cell to obtain the corresponding genetically engineered cells.

A fifth aspect of the present disclosure provides a pharmaceutical composition comprising the fusion protein, the encoding nucleic acid, the recombinant expression vector, and/or the recombinant host cell, and optionally a pharmaceutically acceptable excipient. The present disclosure does not impose any limitation on the formulation form of the pharmaceutical composition, which may be determined by one skilled in the art according to actual needs.

A sixth aspect of the present disclosure provides the use of the fusion protein, the encoding nucleic acid, the recombinant expression vector, the recombinant host cell, and/or the pharmaceutical composition in the manufacture of a medicament for preventing an infection caused by SARS-CoV-2, its variant strains, or other ACE2-dependent coronaviruses. The fusion protein, encoding nucleic acid, recombinant expression vector, recombinant host cell, and/or pharmaceutical composition is suitable for preventing and/or treating an infection caused by SARS-CoV-2, its variant strains, or other ACE2-dependent coronaviruses.

A seventh aspect of the present disclosure provides a method for preventing and/or treating an infection caused by SARS-CoV-2, its variant strains, or other ACE2-dependent coronaviruses, comprising administering to a subject a prophylactically and/or therapeutically effective amount of the fusion protein, the encoding nucleic acid, the recombinant expression vector, the recombinant host cell, and/or the pharmaceutical composition.

**Pseudovirus Preparation**

In the present disclosure, pseudoviruses may be prepared using methods known in the art. For example, as previously described (L. Lu, Q. Liu, Y. Zhu, K.-H. Chan, L. Qin, Y. Li, Q. Wang, J. F.-W. Chan, L. Du, F. Yu, C. Ma, S. Ye, K.-Y. Yuen, R. Zhang, S. Jiang, Structure-based discovery of Middle East respiratory syndrome coronavirus fusion inhibitor. Nat. Commun. 5, 3067 (2014); or Xing L., et al. A five-helix-based SARS-CoV-2 fusion inhibitor targeting Heptad Repeat 2 domain against SARS-CoV-2 and its variants of concern. Viruses. 2022 Mar 13;14(3):597), pseudoviruses were generated by transfecting 293T cells with plasmids. The plasmids comprise the HIV backbone plasmid pNL4-3.Luc.R⁻.E⁻ and a plasmid expressing the S protein from various SARS-CoV-2 variant strains. The amino acid sequences of these S proteins are well-established, the specific plasmids used for expressing S proteins of different variants are listed in the table below.

Table 1:

| Plasmid expressing S proteins of SARS-CoV-2 variants | Genbank No. |
| --- | --- |
| pcDNA3.1-N501Y-S | OP411027.1 |
| pcDNA3.1-B.1.1.7-S | OM189517.1 |
| pcDNA3.1-B.1.351-S | OQ248296.1 |
| pcDNA3.1-P.1-S | OM189519.1 |
| pcDNA3.1-C.37-S | OX002793.1 |
| pcDNA3.1-B.1.617.2-S | OM858819.1 |
| pcDNA3.1-BA.1-S | OX008556.1 |
| pcDNA3.1-BA.2-S | OX315675.1 |
| pcDNA3.1-BA.2.2-S | OQ901124.1 |
| pcDNA3.1-BA.2.5-S | OR281319.1 |
| pcDNA3.1-BA.2.12.1-S | OQ248368.1 |
| pcDNA3.1-BA.2.75-S | OQ300211.1 |
| pcDNA3.1-BA.3-S | OR266137.1 |
| pcDNA3.1-BA.5-S | OQ248383.1 |
| pcDNA3.1-BA.4.6-S | OQ248384.1 |
| pcDNA3.1-BF.7-S | OP863003.1 |

(continued)

| Plasmid expressing S proteins of SARS-CoV-2 variants | Genbank No. |
|---|---|
| pcDNA3.1-BQ.1.1-S | OQ300192.1 |
| pcDNA3.1-XBB.1-S | OQ214900.1 |

## EXAMPLES

The present disclosure is further illustrated by the following examples; however, these examples, whether individually or in combination, shall not be construed as limiting the scope or embodiments of the disclosure. The scope of the disclosure is defined solely by the following claims. Based on this specification and the general knowledge in the art, the scope defined by the claims will be apparent to those of ordinary skill in the art. Those skilled in the art may make various modifications or changes to the technical solutions of the present disclosure without departing from the spirit and scope thereof, and such modifications and changes are intended to be included within the scope of the present disclosure.

In the following examples, unless specific conditions are indicated, procedures are carried out under conventional conditions or in accordance with the manufacturer's recommendations. Reagents and instruments for which no manufacturer is specified are conventional commercially available products. Numerous specific details are provided in the examples below to better illustrate the present disclosure. It will be understood by those skilled in the art that the disclosure may be practiced without these specific details. In other instances, methods, means, equipment, and steps well known to those skilled in the art are not described in detail so as not to obscure the subject matter of the present disclosure.

The protein materials used in the examples are ACE2-linker-EK1 fusion proteins (specifically designated as AL2E, AL5E, AL6E, and AL7E), which were designed by the inventors based on a newly discovered mechanism. This mechanism involves the secreted ACE2 protein binding to the receptor-binding domain (RBD) of the viral S1 protein, thereby inducing conformational changes in the S2 subunit that expose the HR1 region. This exposure facilitates the subsequent binding of the EK1 polypeptide. These proteins are constructed by linking the ACE2 peptidase domain to the EK1 polypeptide via linkers of varying lengths. Subsequently, the proteins were purified via their C-terminal 8×His tag, and their purities and expected molecular identities were confirmed by gel filtration chromatography and Coomassie Brilliant Blue staining.

## Example 1: Identification of AL2E, AL5E, AL6E, and AL7E Proteins by Gel Filtration Chromatography and Coomassie Brilliant Blue Staining

Expression plasmids encoding the AL2E, AL5E, AL6E, and AL7E genes (expression vector: pFuse-hIgG1-Fc2) were synthesized and supplied by Nanjing GenScript Co., Ltd. Using EZ-Trans transfection reagent (purchased from Life iLab Biotech Co., LTD, Cat. No. PD1311-01), the expression plasmids encoding the AL2E, AL5E, AL6E, and AL7E genes were respectively transfected into Expi 293 suspension cells (purchased from Thermo Fisher) at a density of $2 \times 10^6$ cells/mL, according to the manufacturer's instructions. Five days post-transfection, the cell culture supernatant (containing the protein of interest) was collected by centrifugation and filtered through a 0.45 $\mu$m filter membrane to remove cell debris. Ni-NTA beads (purchased from Smart-Lifesciences, Cat. No. 191024) were then added, and the mixture was incubated on a rotary mixer at 4 °C for 10 hours. The liquid containing the beads was loaded onto a protein purification gravity column (purchased from Beyotime). Then, impurity proteins were washed away with a phosphate buffer containing 10 mM imidazole, and finally, the column was eluted with a phosphate buffer containing 250 mM imidazole to obtain the target proteins. The harvested target proteins were centrifuged and filtered through a 30 kDa ultrafiltration tube (purchased from Merck) to remove imidazole, and the protein storage buffer was completely exchanged to phosphate buffer (pH 7.4). After concentration determination, the protein was aliquoted and stored in a -80 °C freezer for subsequent experiments.

The nucleotide sequence of AL2E is as follows (SEQ ID NO: 8):

```
ATGTCAAGCTCTTCCTGGCTCCTTCTCAGCCTTGTTGCTGTAACTGCTGCTCAGTCCACCATTGAGGAA
CAGGCCAAGACATTTTTGGACAAGTTTAACCACGAAGCCGAAGACCTGTTCTATCAAAGTTCACTTGC
TTCTTGGAATTATAACACCAATATTACTGAAGAGAATGTCCAAAACATGAATAATGCTGGGGACAAA
TGGTCTGCCTTTTTAAAGGAACAGTCCACACTTGCCCAAATGTATCCACTACAAGAAATTCAGAATCT
```

CACAGTCAAGCTTCAGCTGCAGGCTCTTCAGCAAAATGGGTCTTCAGTGCTCTCAGAAGACAAGAGC
AAACGGTTGAACACAATTCTAAATACAATGAGCACCATCTACAGTACTGGAAAAGTTTGTAACCCAG
ATAATCCACAAGAATGCTTATTACTTGAACCAGGTTTGAATGAAATAATGGCAAACAGTTTAGACTAC
AATGAGAGGCTCTGGGCTTGGGAAAGCTGGAGATCTGAGGTCGGCAAGCAGCTGAGGCCATTATATG
AAGAGTATGTGGTCTTGAAAAATGAGATGGCAAGAGCAAATCATTATGAGGACTATGGGGATTATTG
GAGAGGAGACTATGAAGTAAATGGGGTAGATGGCTATGACTACAGCCGCGGCCAGTTGATTGAAGAT
GTGGAACATACCTTTGAAGAGATTAAACCATTATATGAACATCTTCATGCCTATGTGAGGGCAAAGTT
GATGAATGCCTATCCTTCCTATATCAGTCCAATTGGATGCCTCCCTGCTCATTTGCTTGGTGATATGTG
GGGTAGATTTTGGACAAATCTGTACTCTTTGACAGTTCCCTTTGGACAGAAACCAAACATAGATGTTA
CTGATGCAATGGTGGACCAGGCCTGGGATGCACAGAGAATATTCAAGGAGGCCGAGAAGTTCTTTGT
ATCTGTTGGTCTTCCTAATATGACTCAAGGATTCTGGGAAAATTCCATGCTAACGGACCCAGGAAATG
TTCAGAAAGCAGTCTGCCATCCCACAGCTTGGGACCTGGGGAAGGGCGACTTCAGGATCCTTATGTG
CACAAAGGTGACAATGGACGACTTCCTGACAGCTCATCATGAGATGGGGCATATCCAGTATGATATG
GCATATGCTGCACAACCTTTTCTGCTAAGAAATGGAGCTAATGAAGGATTCCATGAAGCTGTTGGGG
AAATCATGTCACTTTCTGCAGCCACACCTAAGCATTTAAAATCCATTGGTCTTCTGTCACCCGATTTTC
AAGAAGACAATGAAACAGAAATCAACTTCCTGCTGAAGCAGGCCCTGACCATTGTTGGGACTCTGCC
ATTTACTTACATGTTAGAGAAGTGGAGGTGGATGGTCTTTAAAGGGGAAATTCCCAAAGACCAGTGG
ATGAAAAGTGGTGGGAGATGAAGCGAGAGATAGTTGGGGTGGTGGAACCTGTGCCCCATGATGAA
ACATACTGTGACCCCGCATCTCTGTTCCATGTTTCTAATGATTACTCATTCATTCGATATTACACAAGG
ACCCTTTACCAATTCCAGTTTCAAGAAGCACTTTGTCAAGCAGCTAAACATGAAGGCCCTCTGCACAA
ATGTGACATCTCAAACTCTACAGAAGCTGGACAGAAACTGTTCAATATGCTGAGGCTTGGAAAATCA
GAACCCTGGACCCTAGCATTGGAAAATGTTGTAGGAGCAAAGAACATGAATGTAAGGCCACTGCTCA
ACTACTTTGAGCCCTTATTTACCTGGCTGAAAGACCAGAACAAGAATTCTTTTGTGGGATGGAGTACC
GACTGGAGTCCATATGCAGACGGAGGAGGAGGAAGCGGAGGAGGAGGATCTAGCCTGGACCAGATC
AACGTGACCTTCCTGGACCTGGAGTACGAGATGAAGAAGCTGGAGGAGGCCATCAAGAAGCTGGAG
GAGAGCTACATCGACCTGAAGGAGCTGCTGGAAGTTCTGTTCCAGGGCCCGCATCACCATCATCACC
ATCACCATTGA

The nucleotide sequence of AL5E is as follows (SEQ ID NO: 9):

ATGTCAAGCTCTTCCTGGCTCCTTCTCAGCCTTGTTGCTGTAACTGCTGCTCAGTCCACCATTGAGGAA
CAGGCCAAGACATTTTTGGACAAGTTTAACCACGAAGCCGAAGACCTGTTCTATCAAAGTTCACTTGC
TTCTTGGAATTATAACACCAATATTACTGAAGAGAATGTCCAAAACATGAATAATGCTGGGGACAAA
TGGTCTGCCTTTTTAAAGGAACAGTCCACACTTGCCCAAATGTATCCACTACAAGAAATTCAGAATCT
CACAGTCAAGCTTCAGCTGCAGGCTCTTCAGCAAAATGGGTCTTCAGTGCTCTCAGAAGACAAGAGC
AAACGGTTGAACACAATTCTAAATACAATGAGCACCATCTACAGTACTGGAAAAGTTTGTAACCCAG
ATAATCCACAAGAATGCTTATTACTTGAACCAGGTTTGAATGAAATAATGGCAAACAGTTTAGACTAC
AATGAGAGGCTCTGGGCTTGGGAAAGCTGGAGATCTGAGGTCGGCAAGCAGCTGAGGCCATTATATG
AAGAGTATGTGGTCTTGAAAAATGAGATGGCAAGAGCAAATCATTATGAGGACTATGGGGATTATTG
GAGAGGAGACTATGAAGTAAATGGGGTAGATGGCTATGACTACAGCCGCGGCCAGTTGATTGAAGAT
GTGGAACATACCTTTGAAGAGATTAAACCATTATATGAACATCTTCATGCCTATGTGAGGGCAAAGTT
GATGAATGCCTATCCTTCCTATATCAGTCCAATTGGATGCCTCCCTGCTCATTTGCTTGGTGATATGTG
GGGTAGATTTTGGACAAATCTGTACTCTTTGACAGTTCCCTTTGGACAGAAACCAAACATAGATGTTA
CTGATGCAATGGTGGACCAGGCCTGGGATGCACAGAGAATATTCAAGGAGGCCGAGAAGTTCTTTGT
ATCTGTTGGTCTTCCTAATATGACTCAAGGATTCTGGGAAAATTCCATGCTAACGGACCCAGGAAATG
TTCAGAAAGCAGTCTGCCATCCCACAGCTTGGGACCTGGGGAAGGGCGACTTCAGGATCCTTATGTG
CACAAAGGTGACAATGGACGACTTCCTGACAGCTCATCATGAGATGGGGCATATCCAGTATGATATG
GCATATGCTGCACAACCTTTTCTGCTAAGAAATGGAGCTAATGAAGGATTCCATGAAGCTGTTGGGG
AAATCATGTCACTTTCTGCAGCCACACCTAAGCATTTAAAATCCATTGGTCTTCTGTCACCCGATTTTC
AAGAAGACAATGAAACAGAAATCAACTTCCTGCTGAAGCAGGCCCTGACCATTGTTGGGACTCTGCC
ATTTACTTACATGTTAGAGAAGTGGAGGTGGATGGTCTTTAAAGGGGAAATTCCCAAAGACCAGTGG
ATGAAAAGTGGTGGGAGATGAAGCGAGAGATAGTTGGGGTGGTGGAACCTGTGCCCCATGATGAA
ACATACTGTGACCCCGCATCTCTGTTCCATGTTTCTAATGATTACTCATTCATTCGATATTACACAAGG
ACCCTTTACCAATTCCAGTTTCAAGAAGCACTTTGTCAAGCAGCTAAACATGAAGGCCCTCTGCACAA
ATGTGACATCTCAAACTCTACAGAAGCTGGACAGAAACTGTTCAATATGCTGAGGCTTGGAAAATCA
GAACCCTGGACCCTAGCATTGGAAAATGTTGTAGGAGCAAAGAACATGAATGTAAGGCCACTGCTCA
ACTACTTTGAGCCCTTATTTACCTGGCTGAAAGACCAGAACAAGAATTCTTTTGTGGGATGGAGTACC
GACTGGAGTCCATATGCAGACGGAGGAGGAGGAAGCGGAGGAGGAGGATCTGGAGGAGGAGGAAG

CGGAGGAGGAGGATCTGGAGGAGGAGGAAGCAGCCTGGACCAGATCAACGTGACCTTCCTGGACCT
GGAGTACGAGATGAAGAAGCTGGAGGAGGCCATCAAGAAGCTGGAGGAGAGCTACATCGACCTGAA
GGAGCTGCTGGAAGTTCTGTTCCAGGGCCCGCATCACCATCATCACCATCACCATTGA

The nucleotide sequence of AL6E is as follows (SEQ ID NO: 10):

ATGTCAAGCTCTTCCTGGCTCCTTCTCAGCCTTGTTGCTGTAACTGCTGCTCAGTCCACCATTGAGGAA
CAGGCCAAGACATTTTTGGACAAGTTTAACCACGAAGCCGAAGACCTGTTCTATCAAAGTTCACTTGC
TTCTTGGAATTATAACACCAATATTACTGAAGAGAATGTCCAAAACATGAATAATGCTGGGGACAAA
TGGTCTGCCTTTTTAAAGGAACAGTCCACACTTGCCCAAATGTATCCACTACAAGAAATTCAGAATCT
CACAGTCAAGCTTCAGCTGCAGGCTCTTCAGCAAAATGGGTCTTCAGTGCTCTCAGAAGACAAGAGC
AAACGGTTGAACACAATTCTAAATACAATGAGCACCATCTACAGTACTGGAAAAGTTTGTAACCCAG
ATAATCCACAAGAATGCTTATTACTTGAACCAGGTTTGAATGAAATAATGGCAAACAGTTTAGACTAC
AATGAGAGGCTCTGGGCTTGGGAAAGCTGGAGATCTGAGGTCGGCAAGCAGCTGAGGCCATTATATG
AAGAGTATGTGGTCTTGAAAAATGAGATGGCAAGAGCAAATCATTATGAGGACTATGGGGATTATTG
GAGAGGAGACTATGAAGTAAATGGGGTAGATGGCTATGACTACAGCCGCGGCCAGTTGATTGAAGAT
GTGGAACATACCTTTGAAGAGATTAAACCATTATATGAACATCTTCATGCCTATGTGAGGGCAAAGTT
GATGAATGCCTATCCTTCCTATATCAGTCCAATTGGATGCCTCCCTGCTCATTTGCTTGGTGATATGTG
GGGTAGATTTTGGACAAATCTGTACTCTTTGACAGTTCCCTTTGGACAGAAACCAAACATAGATGTTA
CTGATGCAATGGTGGACCAGGCCTGGGATGCACAGAGAATATTCAAGGAGGCCGAGAAGTTCTTTGT
ATCTGTTGGTCTTCCTAATATGACTCAAGGATTCTGGGAAAATTCCATGCTAACGGACCCAGGAAATG
TTCAGAAAGCAGTCTGCCATCCCACAGCTTGGGACCTGGGGAAGGGCGACTTCAGGATCCTTATGTG
CACAAAGGTGACAATGGACGACTTCCTGACAGCTCATCATGAGATGGGGCATATCCAGTATGATATG
GCATATGCTGCACAACCTTTTCTGCTAAGAAATGGAGCTAATGAAGGATTCCATGAAGCTGTTGGGG
AAATCATGTCACTTTCTGCAGCCACACCTAAGCATTTAAAATCCATTGGTCTTCTGTCACCCGATTTTC
AAGAAGACAATGAAACAGAAATCAACTTCCTGCTGAAGCAGGCCCTGACCATTGTTGGGACTCTGCC
ATTTACTTACATGTTAGAGAAGTGGAGGTGGATGGTCTTTAAAGGGGAAATTCCCAAAGACCAGTGG
ATGAAAAAGTGGTGGGAGATGAAGCGAGAGATAGTTGGGGTGGTGGAACCTGTGCCCCATGATGAA
ACATACTGTGACCCCGCATCTCTGTTCCATGTTTCTAATGATTACTCATTCATTCGATATTACACAAGG
ACCCTTTACCAATTCCAGTTTCAAGAAGCACTTTGTCAAGCAGCTAAACATGAAGGCCCTCTGCACAA
ATGTGACATCTCAAACTCTACAGAAGCTGGACAGAAACTGTTCAATATGCTGAGGCTTGGAAAATCA
GAACCCTGGACCCTAGCATTGGAAAATGTTGTAGGAGCAAAGAACATGAATGTAAGGCCACTGCTCA
ACTACTTTGAGCCCTTATTTACCTGGCTGAAAGACCAGAACAAGAATTCTTTTGTGGGATGGAGTACC
GACTGGAGTCCATATGCAGACGGAGGAGGAGGAAGCGGAGGAGGAGGAAGCGGAGGAGGAGGATC
TGGAGGAGGAGGAAGCGGAGGAGGAGGATCTGGAGGAGGAGGAAGCAGCCTGGACCAGATCAACG
TGACCTTCCTGGACCTGGAGTACGAGATGAAGAAGCTGGAGGAGGCCATCAAGAAGCTGGAGGAGA
GCTACATCGACCTGAAGGAGCTGCTGGAAGTTCTGTTCCAGGGCCCGCATCACCATCATCACCATCAC
CATTGA

The nucleotide sequence of AL7E is as follows (SEQ ID NO: 11):

ATGTCAAGCTCTTCCTGGCTCCTTCTCAGCCTTGTTGCTGTAACTGCTGCTCAGTCCACCATTGAGGAA
CAGGCCAAGACATTTTTGGACAAGTTTAACCACGAAGCCGAAGACCTGTTCTATCAAAGTTCACTTGC
TTCTTGGAATTATAACACCAATATTACTGAAGAGAATGTCCAAAACATGAATAATGCTGGGGACAAA
TGGTCTGCCTTTTTAAAGGAACAGTCCACACTTGCCCAAATGTATCCACTACAAGAAATTCAGAATCT
CACAGTCAAGCTTCAGCTGCAGGCTCTTCAGCAAAATGGGTCTTCAGTGCTCTCAGAAGACAAGAGC
AAACGGTTGAACACAATTCTAAATACAATGAGCACCATCTACAGTACTGGAAAAGTTTGTAACCCAG
ATAATCCACAAGAATGCTTATTACTTGAACCAGGTTTGAATGAAATAATGGCAAACAGTTTAGACTAC
AATGAGAGGCTCTGGGCTTGGGAAAGCTGGAGATCTGAGGTCGGCAAGCAGCTGAGGCCATTATATG
AAGAGTATGTGGTCTTGAAAAATGAGATGGCAAGAGCAAATCATTATGAGGACTATGGGGATTATTG
GAGAGGAGACTATGAAGTAAATGGGGTAGATGGCTATGACTACAGCCGCGGCCAGTTGATTGAAGAT
GTGGAACATACCTTTGAAGAGATTAAACCATTATATGAACATCTTCATGCCTATGTGAGGGCAAAGTT
GATGAATGCCTATCCTTCCTATATCAGTCCAATTGGATGCCTCCCTGCTCATTTGCTTGGTGATATGTG
GGGTAGATTTTGGACAAATCTGTACTCTTTGACAGTTCCCTTTGGACAGAAACCAAACATAGATGTTA
CTGATGCAATGGTGGACCAGGCCTGGGATGCACAGAGAATATTCAAGGAGGCCGAGAAGTTCTTTGT
ATCTGTTGGTCTTCCTAATATGACTCAAGGATTCTGGGAAAATTCCATGCTAACGGACCCAGGAAATG
TTCAGAAAGCAGTCTGCCATCCCACAGCTTGGGACCTGGGGAAGGGCGACTTCAGGATCCTTATGTG
CACAAAGGTGACAATGGACGACTTCCTGACAGCTCATCATGAGATGGGGCATATCCAGTATGATATG
GCATATGCTGCACAACCTTTTCTGCTAAGAAATGGAGCTAATGAAGGATTCCATGAAGCTGTTGGGG
AAATCATGTCACTTTCTGCAGCCACACCTAAGCATTTAAAATCCATTGGTCTTCTGTCACCCGATTTTC

AAGAAGACAATGAAACAGAAATCAACTTCCTGCTGAAGCAGGCCCTGACCATTGTTGGGACTCTGCC
ATTTACTTACATGTTAGAGAAGTGGAGGTGGATGGTCTTTAAAGGGGAAATTCCCAAAGACCAGTGG
ATGAAAAAGTGGTGGGAGATGAAGCGAGAGATAGTTGGGGTGGTGGAACCTGTGCCCCATGATGAA
ACATACTGTGACCCCGCATCTCTGTTCCATGTTCTAATGATTACTCATTCATTCGATATTACACAAGG
ACCCTTTACCAATTCCAGTTTCAAGAAGCACTTTGTCAAGCAGCTAAACATGAAGGCCCTCTGCACAA
ATGTGACATCTCAAACTCTACAGAAGCTGGACAGAAACTGTTCAATATGCTGAGGCTTGGAAAATCA
GAACCCTGGACCCTAGCATTGGAAAATGTTGTAGGAGCAAAGAACATGAATGTAAGGCCACTGCTCA
ACTACTTTGAGCCCTTATTTACCTGGCTGAAAGACCAGAACAAGAATTCTTTTGTGGGATGGAGTACC
GACTGGAGTCCATATGCAGACGGAGGAGGAGGAAGCGGAGGAGGAGGAAGCGGAGGAGGAGGAAG
CGGAGGAGGAGGATCTGGAGGAGGAGGAAGCGGAGGAGGAGGATCTGGAGGAGGAGGAAGCAGCC
TGGACCAGATCAACGTGACCTTCCTGGACCTGGAGTACGAGATGAAGAAGCTGGAGGAGGCCATCAA
GAAGCTGGAGGAGAGCTACATCGACCTGAAGGAGCTGCTGGAAGTTCTGTTCCAGGGCCCGCATCAC
CATCATCACCATCACCATTGA

The AL2E, AL5E, AL6E, and AL7E proteins, initially purified by affinity chromatography, were further purified using a Superose 6 Increase 10/300 GL gel filtration chromatography column (GE Healthcare), and the purified proteins were buffer-exchanged into PBS buffer. Specifically, a Superose 6 Increase 10/300 GL gel filtration chromatography column (GE Healthcare) was installed on an ÄKTA avant protein purification system (GE Healthcare). The flow rate was set to 0.8 mL/min, the column pressure limit was set to 1.5 MPa, and the column was washed with 48 mL of PBS buffer. Subsequently, 0.5 mL of each of the AL2E, AL5E, AL6E, and AL7E protein solutions at 5 mg/mL was injected into the sample loop via the injection port of the instrument. The method was then programmed for sample loop loading with a flow rate of 0.8 mL/min, a column pressure limit of 1.5 MPa, and an elution volume of 24 mL. Automatic fraction collection was initiated upon detection of absorbance at 280 nm, and the run was started. The same procedure was applied to each of the AL5E, AL6E, and AL7E proteins. Through the process described above, the protein storage buffer was automatically exchanged to PBS buffer. SDS-PAGE analysis was performed on the AL2E, AL5E, AL6E, and AL7E proteins. Specifically, 10 $\mu$L of 5$\times$ SDS-PAGE protein loading buffer (Yeasen Biotechnology, Cat. No. 20315ES20) was added to 40 $\mu$L of protein solution. The mixture was heated at 100 °C for 10 minutes and then cooled on ice. A 20 $\mu$L aliquot of the sample was loaded into the wells of an SDS-PAGE gel. Electrophoresis was performed at 80 V for 20 minutes, after which the voltage was increased to 120 V and allowed to run for 40 minutes. After electrophoresis, the gel was removed from the cassette and stained with protein staining solution (BioSharp, Cat. No. 243183). Upon completion of staining, the results were photographed and saved.

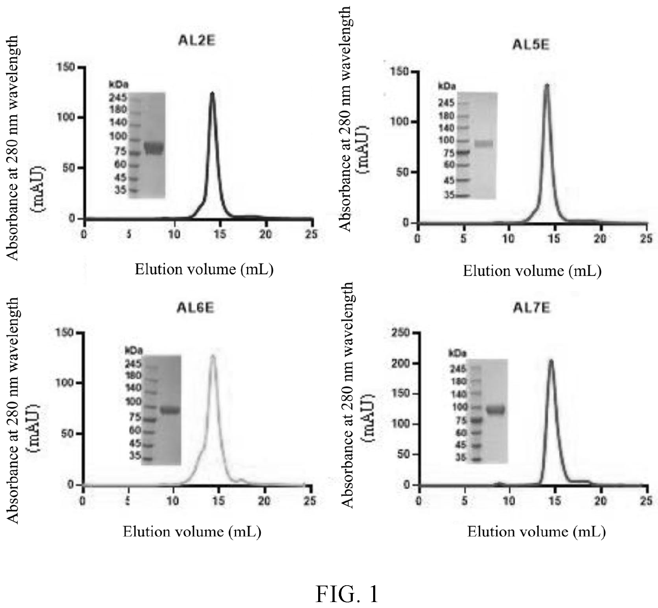

**Results:** The elution peaks of the four proteins in gel filtration chromatography were single and symmetrical, indicating good homogeneity in solution. SDS-PAGE analysis showed that the apparent molecular weights of the fusion proteins AL2E, AL5E, AL6E, and AL7E matched their theoretical molecular weights, confirming correct expression of the fusion proteins. Furthermore, the purity exceeded 99%, making them suitable for subsequent experiments. The gel filtration chromatography profiles and SDS-PAGE results are shown in FIG. 1.

## Example 2: Screening of Inhibition by AL2E, AL5E, AL6E, and AL7E Proteins Against Pseudoviruses of SARS-CoV-2 VOCs and Omicron Variant Strains

Preparation of pseudoviruses for SARS-CoV-2 VOCs and Omicron variant strains (Reference: Xing L., et al. A five-helix-based SARS-CoV-2 fusion inhibitor targeting Heptad Repeat 2 domain against SARS-CoV-2 and its variants of concern. Viruses. 2022 Mar 13;14(3):597): Plasmids encoding the S protein genes of SARS-CoV-2 VOCs or Omicron variant strains (plasmid name: pcDNA 3.1, synthesized by BGI TECH SOLUTIONS (BEIJING LIUHE) CO., LIMITED) were co-transfected with the pNL4-3.Luc.RE plasmid (containing the luciferase expression gene, purchased from Addgene) into HEK293T cells (purchased from Thermo Fisher) at a mass ratio of 1:2, 24 hours after cell passaging. 10 hours post-transfection, the original culture medium was discarded and replaced with fresh DMEM containing 10% serum (purchased from Gibco, Cat. No. C12430500BT). 48 hours after the medium exchange, the cell culture supernatant was collected. After centrifugation at 3000 rpm to remove cell debris, the corresponding pseudoviruses were obtained (as shown in Table 2), aliquoted, and stored at -80 °C for later use.

Human colon cancer cells (Caco-2) expressing the ACE2 receptor were seeded into 96-well plates at a density of $10^4$ cells/well and cultured at 37 °C. In 96-well U-bottom plates, each of the AL2E, AL5E, AL6E, and AL7E proteins was serially diluted 5-fold using DMEM medium, resulting in a concentration range from 100 nM to 0.0064 nM. Subsequently, pseudoviruses of SARS-CoV-2 VOCs or Omicron variant strains with equal titers and volumes were added. The mixtures were incubated at 37 °C for 1 hour. The original medium in the cell plates was discarded, and 100 $\mu$L of the fusion protein/pseudovirus mixture (drug-treated wells) or pseudovirus alone (virus wells) was added to the target cells after removing the supernatant. Treatment groups without pseudovirus served as cell control wells (cell wells). After culturing at 37 °C for 12 hours, the medium was replaced with fresh DMEM containing 10% serum. After 72 hours, the original medium in the 96-well cell plates was discarded, and 40 $\mu$L of cell lysis buffer (Promega, Cat. No. E1531) was added to each well.

The plates were shaken on an orbital shaker for 45 minutes to lyse the cells. Then, 30 μL of cell lysate from each well was transferred to a 96-well ELISA plate, and 30 μL of firefly luciferase substrate (purchased from Promega, Cat. No. E1501) was added to each well. The ELISA plates were placed in a microplate reader (purchased from PerkinElmer) to measure the fluorescence value of each well. The inhibition rate curves were plotted, and the half-maximal inhibitory concentration ($IC_{50}$) of each protein was calculated. Specifically, the virus inhibition rate for each drug-treated well was calculated using the following formula: Inhibition Rate = (fluorescence intensity value of a virus well - fluorescence intensity value of a drug-treated well) / (fluorescence intensity value of a virus well - fluorescence intensity value of a cell well) × 100%, in which the virus well did not include fusion protein treatment, the drug-treated well included fusion protein treatment, and the cell well did not include virus treatment. The calculated virus inhibition rates for each well were imported into GraphPad Prism software. The inhibition rate curves and $IC_{50}$ values were generated by selecting the Variable Slope option under the Dose-Response-Inhibition mode.

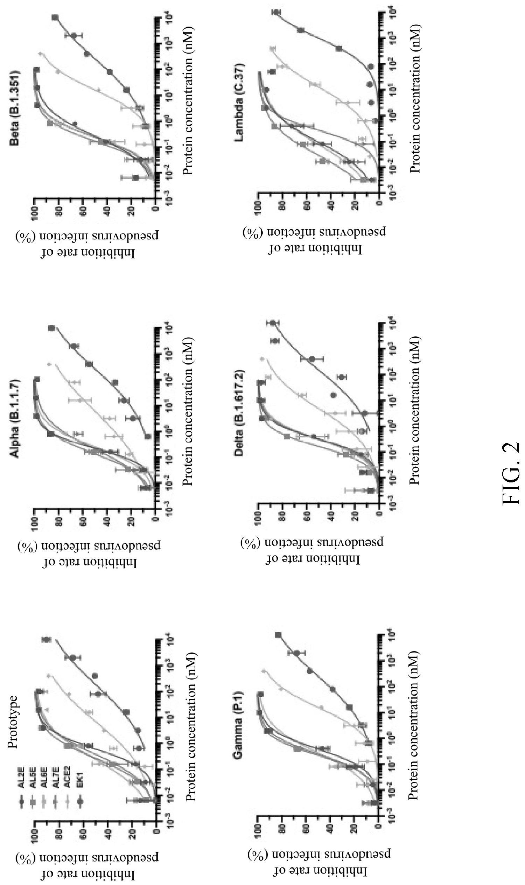

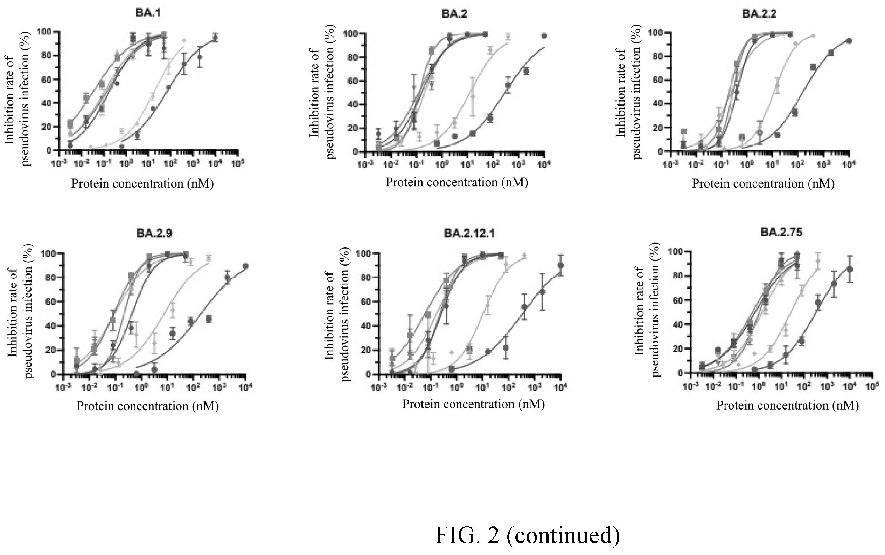

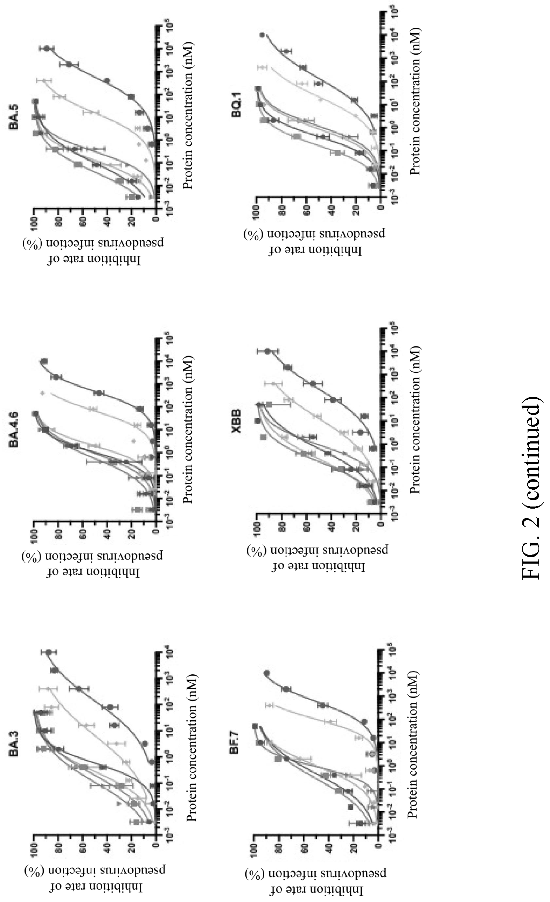

The resulting viral inhibition curves and calculated $IC_{50}$ values are shown in FIG. 2 and Table 2, respectively. The $IC_{50}$ of the AL2E protein against various pseudoviruses ranged from 0.09 to 1.03 nM; the $IC_{50}$ of the AL5E protein ranged from 0.03 to 0.93 nM; the $IC_{50}$ of the AL6E protein ranged from 0.07 to 1.96 nM; and the $IC_{50}$ of the AL7E protein ranged from 0.11 to 1.14 nM. Unexpectedly, the results indicated that the proteins with different linker lengths, the AL5E protein exhibited the best inhibitory effect against various pseudoviruses, which was 27- to 600-fold higher than that of the ACE2 protein alone. Furthermore, despite viral variations, the $IC_{50}$ values of the AL5E protein against various pseudoviruses showed no significant difference, indicating that the AL5E protein possesses broad-spectrum inhibitory activity against all variant strains.

Table 2: Inhibitory Activity of AL2E, AL5E, AL6E, and AL7E Proteins Against Pseudoviruses of SARS-CoV-2 VOCs and Omicron Variant Strains

| | ACE2-EK1 protein/ pseudovirus type | ALSE | AL6E | AL7E | ACE2 | EK1 | Fold enhancement of AL5E over ACE2 |
|---|---|---|---|---|---|---|---|
| | prototype (prototype) | 0.60 | 0.26 | 0.23 | 0.37 | 7.00 | 185.90 | 26.92 |
| | Alpha | 0.22 | 0.13 | 0.25 | 0.21 | 6.20 | 268.10 | 47.69 |
| | Beta | 0.27 | 0.17 | 0.25 | 0.24 | 16.06 | 257.00 | 94.47 |
| | Gamma | 0.39 | 0.22 | 0.28 | 0.25 | 15.06 | 277.00 | 68.45 |
| | Delta | 0.32 | 0.16 | 0.31 | 0.31 | 4.85 | 150.50 | 30.31 |
| | Lambda | 0.09 | 0.03 | 0.07 | 0.27 | 12.82 | 994.90 | 427.33 |
| | BA.1 | 0.18 | 0.03 | 0.11 | 0.14 | 18.73 | 70.50 | 624.33 |
| $IC_{50}$ (nM) | BA.2 | 0.16 | 0.13 | 0.21 | 0.12 | 12.59 | 297.40 | 96.85 |
| | BA.2.2 | 0.40 | 0.20 | 0.24 | 0.28 | 11.86 | 162.30 | 59.30 |
| | BA.2.9 | 0.43 | 0.08 | 0.09 | 0.21 | 7.63 | 198.60 | 95.38 |
| | BA.2.12.1 | 0.26 | 0.06 | 0.17 | 0.23 | 10.59 | 342.10 | 176.50 |
| | BA.2.75 | 0.69 | 0.52 | 1.40 | 1.04 | 26.54 | 331.40 | 51.04 |
| | BA.3 | 0.58 | 0.20 | 0.27 | 0.11 | 6.51 | 136.30 | 32.55 |
| | BA.4.6 | 1.03 | 0.93 | 1.96 | 0.56 | 65.86 | 473.30 | 70.82 |
| | BA.5 | 0.10 | 0.04 | 0.13 | 0.31 | 15.27 | 568.30 | 381.75 |
| | BF.7 | 0.46 | 0.28 | 1.01 | 0.93 | 101.40 | 583.50 | 362.14 |
| | XBB | 0.64 | 0.17 | 0.23 | 0.94 1.14 | 12.66 | 233.00 | 74.47 |
| | BQ.1 | 0.40 | 0.16 | 0.86 | 1.14 | 21.79 | 135.80 | 136.19 |

**Example 3: Inhibition of Live Omicron BA.2.2 Infection by the AL5E Protein**

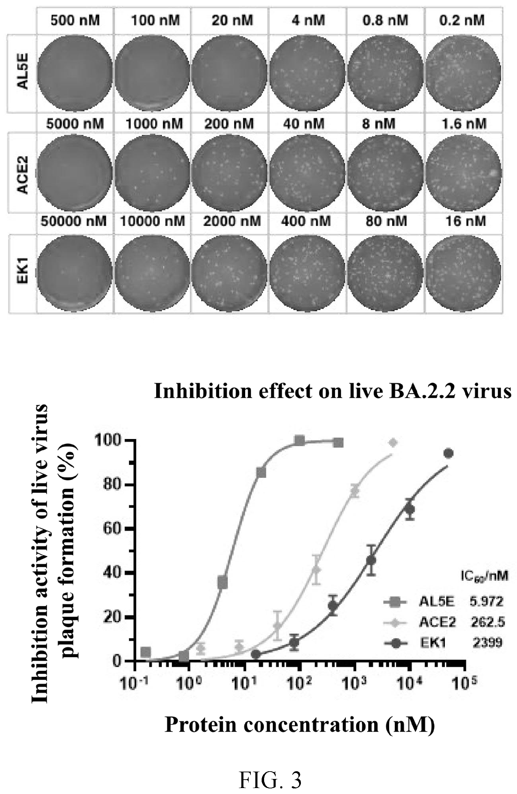

African green monkey kidney cells (Vero-E6) were seeded into a 96-well plate at a density of $1.6 \times 10^4$ cells/well and cultured at 37 °C for 12 hours. In the 96-well U-bottom plate, the AL5E protein was serially diluted 5-fold in DMEM medium, with concentrations ranging from 0.2 nM to 500 nM; the ACE2 protein was serially diluted 5-fold, with concentrations ranging from 1.6 nM to 5000 nM; and the EK1 polypeptide was serially diluted 5-fold, with concentrations ranging from 16 nM to 50,000 nM. An equal volume of live SARS-CoV-2 BA.2.2 virus (100 $TCID_{50}$) was then added. This experiment was conducted in the P3 Laboratory of Shanghai Medical College, Fudan University, and the virus was provided by the aforementioned institution. The mixtures were incubated at 37 °C for 1 hour. The original medium in the cell plate was discarded, and 100 μL of the AL5E protein/virus mixture, ACE2 protein/virus mixture, or EK1 polypeptide/virus mixture (drug-treated wells), or live virus alone (virus wells), was added to the target Vero-E6 cells after removing the supernatant. Treatment groups without virus addition served as cell wells. After incubation at 37 °C for 12 hours, the medium was replaced with DMEM containing 1% methylcellulose and 2% FBS. After 72 hours, the medium was discarded, and the cells were fixed with 4% paraformaldehyde. Subsequently, 0.2% Triton X-100 was added to disrupt the cell membranes and inactivate the virus. A blocking solution (3% bovine serum albumin in PBS buffer) was then added, and the plates were blocked at 37 °C for 2 hours. Next, rabbit anti-SARS-CoV-2 N protein antibody (purchased from Sino Biological Inc., Cat. No. 40588-T62), diluted 1:1500, was added and incubated at 37 °C for 2 hours. After three washes with PBS, FITC-labeled goat anti-rabbit antibody (purchased from Thermo Fisher Scientific, Cat. No. A32731), diluted 1:2000, was added and incubated at 37 °C for 1 hour. After four washes with PBS, the number of fluorescent cells per well was determined using a CTL ImmunoSpot analyzer. The virus inhibition rate for each drug-treated well was calculated using the following formula: Inhibition Rate = (fluorescent cell number of a virus well - fluorescent cell number of a drug-treated well) / (fluorescent cell number of a virus well - fluorescent cell number of a cell well) $\times$ 100%, in which the virus well did not include fusion protein treatment, the drug-treated well included fusion protein treatment, and the cell well did not include virus treatment. The calculated virus inhibition rates for each well were imported into GraphPad Prism software. The inhibition rate curves and $IC_{50}$ values were generated by selecting the Variable Slope option under the Dose-Response-Inhibition mode. The resulting viral inhibition curves and calculated $IC_{50}$ values are shown in FIG. 3. Calculations showed that the $IC_{50}$ of the AL5E protein against the live BA.2.2 virus was 5.972 nM, and its inhibitory effect was 44-fold stronger than that of the ACE2 protein alone ($IC_{50}$ = 262.5 nM). These results indicate that the AL5E protein exhibits potent inhibitory effects in both pseudovirus and live virus cell infection models.

## Example 4: Inactivation of SARS-CoV-2 VOCs and Omicron Variant Pseudoviruses by AL5E Protein

The experiment regarding the inactivation of SARS-CoV-2 VOCs and Omicron variant pseudoviruses by the AL5E protein was performed with reference to: Wang X., et al. Synergistic effect by combining a gp120-binding protein and a gp41-binding antibody to inactivate HIV-1 virions and inhibit HIV-1 infection. Molecules. 2021 Mar 31;26(7):1964.

Specifically, human colon cancer cells (Caco-2) expressing the ACE2 receptor were seeded into a 96-well plate at a density of $10^4$ cells/well and cultured at 37 °C for 24 hours. In EP tubes, each of the AL2E, AL5E, AL6E, and AL7E proteins was serially diluted 4-fold in DMEM medium, with concentrations ranging from 500 nM to 0.488 nM. Then, pseudoviruses of SARS-CoV-2 VOCs and Omicron variant strains (prepared as described in Example 2) with equal titers and volumes were added. The mixtures were incubated at 4 °C for 1 hour. PEG-6000 was added to the protein/pseudovirus mixtures or to the pseudovirus-only controls to a final concentration of 10% to precipitate the virus. Groups without virus addition served as cell controls. Subsequently, centrifugation was performed at 12,000 rpm for 1 hour at 4 °C. The supernatant was discarded, and 3% PEG-6000 (diluted in 10 mg/mL BSA) was added to wash away proteins remaining on the virus surface. After three washes, the virus was resuspended in DMEM and added to the Caco-2 target cells after removing the supernatant. After incubation at 37 °C for 12 hours, the medium was replaced with fresh DMEM containing 10% FBS. At 72 hours, luciferase activity was measured. The assay method was the same as in Example 2. Specifically, the virus inactivation rate for each drug-treated well was calculated using the following formula:

Inactivation rate = (fluorescent cell number of a virus well- fluorescent cell number of a drug-treated well) / (fluorescent cell number of a virus well- fluorescent cell number of a cell well) $\times$ 100%. The calculated viral inactivation rates for each well were imported into GraphPad Prism software. The inactivation rate curves and half-maximal effective concentration ($EC_{50}$) values were generated by selecting the Variable Slope option under the Dose-Response - Inhibition mode.

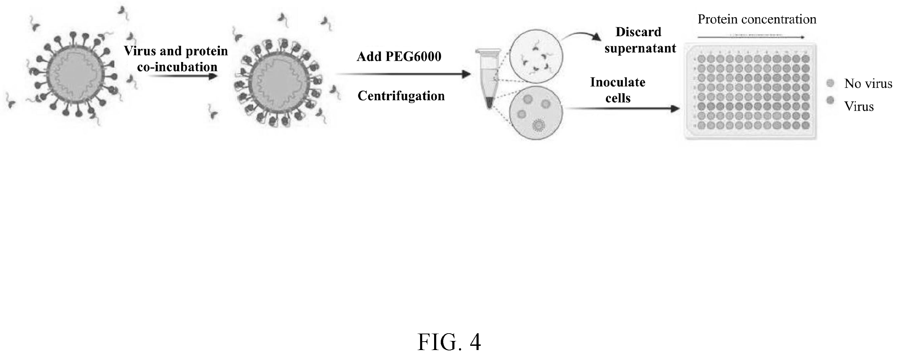

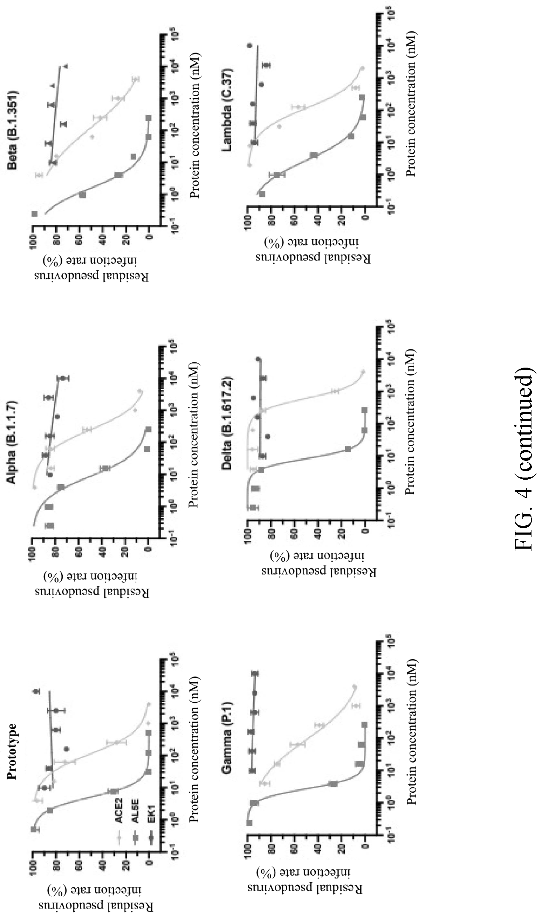

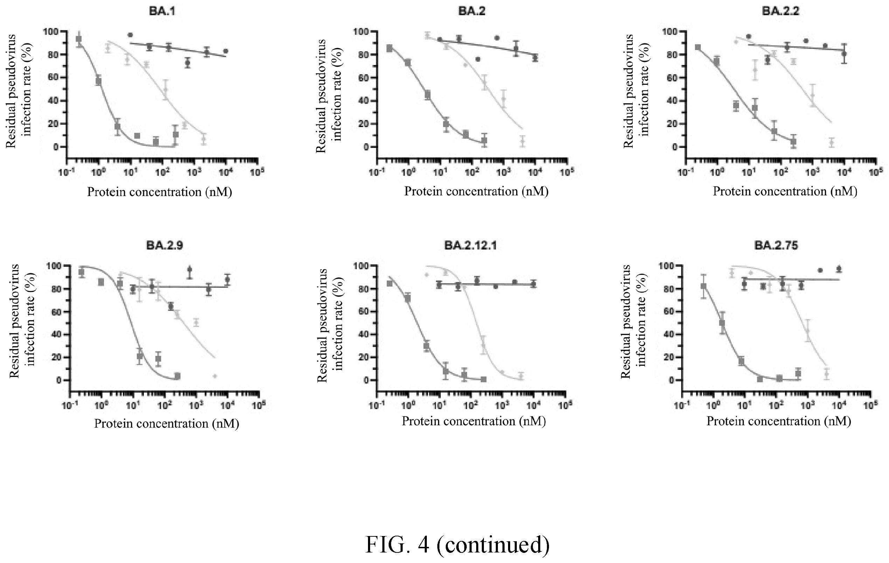

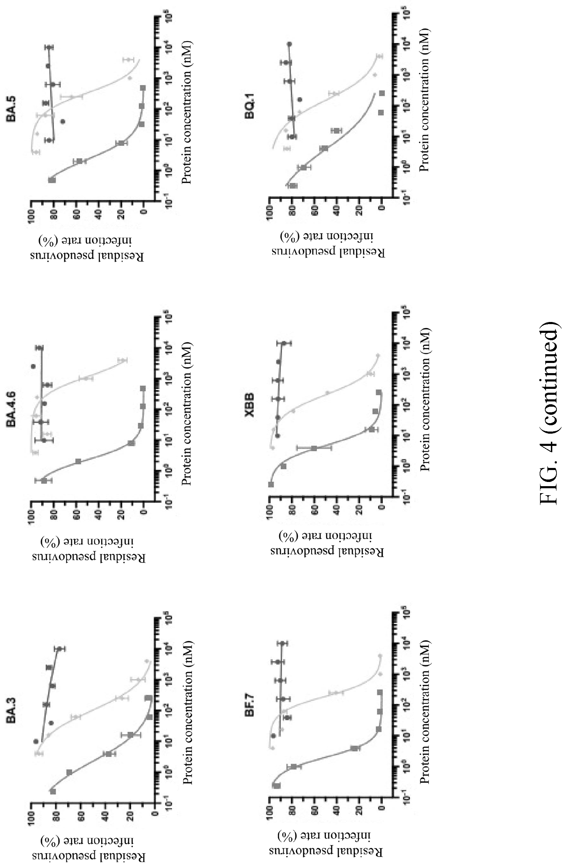

The resulting viral inactivation curves and calculated $EC_{50}$ values are shown in FIG. 4 and Table 3, respectively. The $EC_{50}$ of the AL5E protein against various pseudoviruses ranged from 1.29 to 8.72 nM, and its virus inactivation effect was 22- to 507-fold greater than that of the ACE2 protein alone. Furthermore, despite viral variations, the $EC_{50}$ values of the AL5E protein against various pseudoviruses showed no significant difference, indicating that the protein possesses broad-spectrum inactivation activity against all variant strains.

Table 3: Inactivation Activity of AL5E Protein Against Pseudoviruses of SARS-CoV-2 VOCs and Omicron Variant Strains

| ACE2-EK1 protein/pseudovirus type | AL5E | ACE2 | Fold enhancement of AL5E over ACE2 |
|---|---|---|---|
| Prototype | 5.10 | 113.50 | 22.25 |
| Alpha | 8.72 | 242.90 | 27.86 |
| Beta | 1.57 | 123.10 | 78.41 |
| Gamma | 2.70 | 88.45 | 32.76 |
| Delta | 8.21 | 605.30 | 73.73 |

(continued)

| ACE2-EK1 protein/pseudovirus type | | AL5E | ACE2 | Fold enhancement of AL5E over ACE2 |
|---|---|---|---|---|
| | Lambda | 2.75 | 115.50 | 42.00 |
| | BA.1 | 1.29 | 82.18 | 63.71 |
| | BA.2 | 3.04 | 329.30 | 108.32 |
| | BA.2.2 | 3.35 | 476.40 | 142.21 |
| EC$_{50}$ (nM) | BA.2.9 | 8.62 | 406.60 | 47.17 |
| | BA.2.12.1 | 1.94 | 76.07 | 39.21 |
| | BA.2.75 | 1.91 | 677.00 | 354.45 |
| | BA.3 | 2.26 | 52.13 | 23.07 |
| | BA.4.6 | 2.28 | 1158.00 | 507.89 |
| | BA.5 | 2.27 | 336.30 | 148.15 |
| | BF.7 | 1.99 | 191.70 | 96.33 |
| | XBB | 4.69 | 212.10 | 45.22 |
| | BQ.1 | 3.73 | 149.40 | 40.05 |

**Example 5: Inhibition and Inactivation of Other ACE2-dependent Coronaviruses by AL5E Protein**

Preparation of Pseudoviruses for SARS-CoV, Human Coronavirus NL63, and Bat-Derived SARS-CoV-Like Coronaviruses: Plasmids encoding the S protein genes of SARS-CoV, Human Coronavirus NL63, and bat-derived SARS-CoV-like coronaviruses (WIV1 and Rs3367) (plasmid: pcDNA 3.1, synthesized by BGI TECH SOLUTIONS (BEIJING LIUHE) CO., LIMITED) were co-transfected with the pNL4-3.Luc.RE plasmid (containing the luciferase expression gene, purchased from Addgene) into HEK293T cells (purchased from Thermo Fisher) at a mass ratio of 1:2, 24 hours after cell passaging. 10 hours post-transfection, the original culture medium was discarded and replaced with fresh DMEM containing 10% serum (purchased from Gibco, Cat. No. C12430500BT). 48 hours after the medium exchange, the cell culture supernatant was collected. After centrifugation at 3000 rpm to remove cell debris, the corresponding pseudoviruses were obtained, aliquoted, and stored at -80 °C for later use.

(1) Inhibition of Other ACE2-dependent Coronaviruses by the AL5E protein

Human colon cancer cells (Caco-2) expressing the ACE2 receptor were seeded into a 96-well plate at a density of $10^4$ cells/well and cultured at 37 °C for 24 hours. In the 96-well U-bottom plate, each of the AL2E, AL5E, AL6E, and AL7E proteins was serially diluted 5-fold, resulting in concentrations ranging from 100 nM to 0.0064 nM. Subsequently, the pseudoviruses of SARS-CoV, Human Coronavirus NL63, and bat-derived SARS-CoV-like coronaviruses with equal titers and volumes were added. The remaining procedures were performed as described in Example 2.

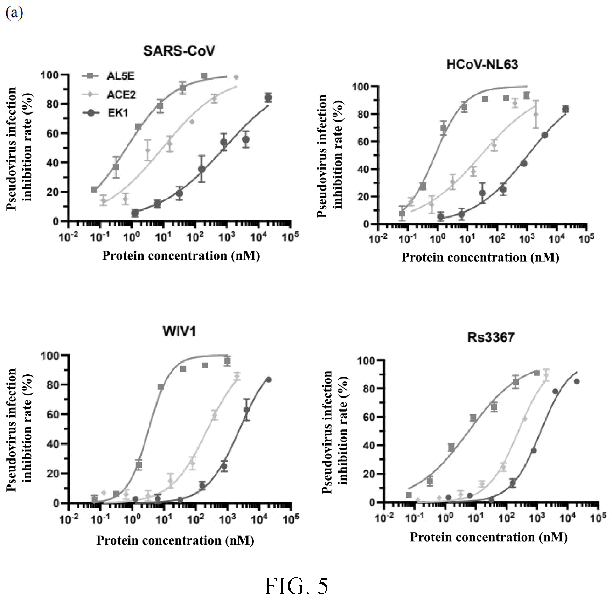

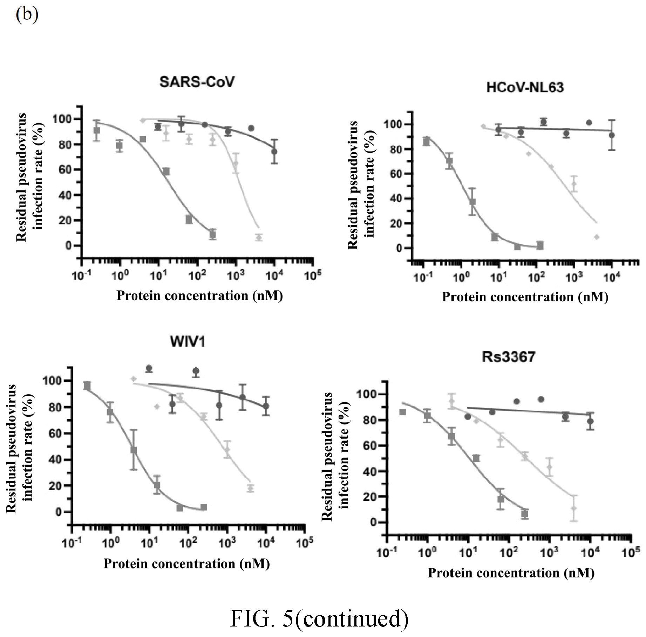

The resulting viral inhibition curves and calculated IC$_{50}$ values are shown in panel (a) of FIG. 5 and Table 4. Calculations showed that the IC$_{50}$ of the AL5E protein against the 4 pseudoviruses ranged from 0.69 to 5.90 nM, and its inhibitory effect was 14- to 70-fold stronger than that of the ACE2 protein alone. These results indicate that the AL5E protein also exhibits potent inhibitory effects against other ACE2-dependent coronaviruses.

Table 4: Inhibition and Inactivation Activities of AL5E Protein Against Other ACE2-dependent Coronaviruses

| ACE2-EK1 protein/pseudovirus type | Inhibitory activity/IC$_{50}$ (nM) | | | |
|---|---|---|---|---|
| | SARS-CoV | HCoV-NL63 | WIV1 | Rs3367 |
| AL5E | 0.69 | 0.83 | 3.36 | 5.90 |
| ACE2 | 9.50 | 28.37 | 239.00 | 249.10 |
| EK1 | 841.40 | 1072.00 | 2448.00 | 1344.00 |
| Fold enhancement in activity of AL5E over ACE2 | 13.80 | 34.37 | 71.22 | 42.23 |
| ACE2- EK1 protein/pseudovirus type | Inactivation activity/EC$_{50}$ (nM) | | | |
| | SARS-CoV | HCoV-NL63 | WIV1 | Rs3367 |
| AL5E | 18.13 | 1.08 | 3.54 | 10.73 |
| ACE2 | 1235.00 | 583.60 | 758.00 | 274.10 |
| Fold enhancement in activity of AL5E over ACE2 | 68.12 | 539.87 | 213.88 | 25.55 |

(2) Inactivation of Other ACE2-dependent Coronaviruses by the AL5E Protein

Human colon cancer cells (Caco-2) expressing the ACE2 receptor were seeded into a 96-well plate at a density of $10^4$ cells/well and cultured at 37 °C for 24 hours. In EP tubes, each of the AL2E, AL5E, AL6E, and AL7E proteins was serially diluted 4-fold, resulting in concentrations ranging from 250 nM to 0.244 nM. Subsequently, the pseudoviruses of SARS-CoV, Human Coronavirus NL63, and bat-derived SARS-CoV-like coronaviruses (WIV1 and Rs3367) with equal titers and volumes were added (the preparation method was as described in Example 5(1)). The remaining procedures were performed as described in Example 2.

The resulting viral inactivation curves and calculated $EC_{50}$ values are shown in panel (b) of FIG. 5 and Table 4. Calculations showed that the $EC_{50}$ of the AL5E protein against the 4 pseudoviruses ranged from 1.08 to 18.13 nM, and its inactivation effect was 26- to 540-fold stronger than that of the ACE2 protein alone. These results indicate that the AL5E protein also maintains potent inactivation effects against other ACE2-dependent coronaviruses. Importantly, compared to ACE2 alone, the AL5E protein exhibited significantly enhanced inactivation efficacy against all 4 pseudoviruses. This suggests that the AL5E protein exhibits superior efficacy in inactivating free viral particles of coronaviruses that have caused past outbreaks, possess re-emergence potential, or pose a significant threat to human health.

## Example 6: Results of AL5E Protein in Preventing and Protecting K18-ACE2 Transgenic Mice from Human Coronavirus NL63 Infection

To determine the efficacy of the AL5E protein in preventing and protecting K18-ACE2 transgenic mice from infection by Human Coronavirus NL63 (provided by National Institute for Viral Disease Control and Prevention, Chinese Center for Disease Control and Prevention), female human ACE2 transgenic mice aged 6-8 weeks were purchased from GemPharmatech Co., Ltd. for viral challenge study. In the prophylactic group, mice were treated with the AL5E protein prepared in Example 1 via pulmonary nebulization at a dose of 1.5 mg/kg body weight, administered 6 hours before the viral challenge (7000 PFU). In the therapeutic group, mice were treated with the AL5E protein via pulmonary nebulization at a dose of 15 mg/kg body weight at 12, 24, 48, 72, and 96 hours post-challenge. 5 days after the viral challenge, the mice were euthanized, and their lungs were excised and photographed to observe organ lesions. Subsequently, the lungs were fixed with 4% paraformaldehyde for tissue sectioning and histological analysis to observe pathological changes (by H&E staining which was performed by Wuhan Servicebio Technology Co., Ltd.). Concurrently, lung tissues were homogenized, and total RNA was extracted to detect the copy number of the viral N gene in the lungs via RT-qPCR. Specifically, RNA extraction was performed using the RNAsimple Total RNA Kit (purchased from TIANGEN, Cat. No. DP419) following the manufacturer's instructions. The extracted RNA was subjected to one-step fluorescent quantitative PCR using the One-Step PrimeScript RT-PCR Kit (Takara, Cat. No. R064A) to quantify the N gene copies. The amplification primers and probe were as follows:

SARS-CoV-2 N-F: 5'- GGGGAACTTCTCCTGCTAGAAT -3';
SARS-CoV-2 N-R: 5'- CAGACATTTTGCTCTCAAGCTG -3'.

The amplification probe was SARS-CoV-2-N-probe. SARS-CoV-2-N-probe was 5'-FAM-TTGCTGCTGCTTG ACAGATT -TAMRA-3'.
These primers and the probe were synthesized by BGI TECH SOLUTIONS (BEIJING LIUHE) CO., LIMITED. The amplification protocol was as follows: 45 °C for 5 minutes; 95 °C for 10 seconds; followed by 30 cycles of 95 °C for 10 seconds, 50 °C for 30 seconds, and 72 °C for 30 seconds, steps 3-5 cycled 30 times.

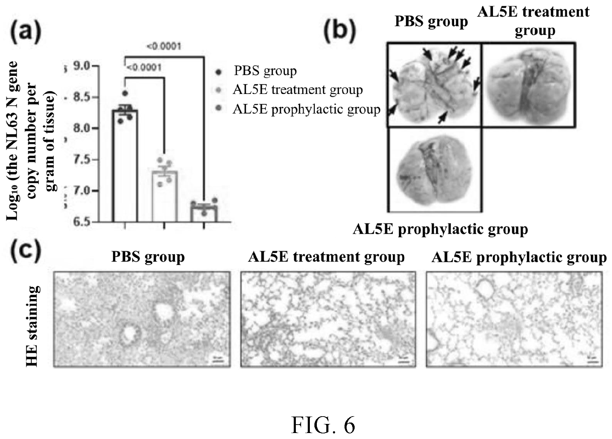

Statistical analysis was performed using GraphPad Prism 8.0. Comparisons among multiple groups were conducted using one-way ANOVA, followed by Tukey's post-hoc test for multiple comparisons. A P-value < 0.05 indicated a significant difference, and a P-value < 0.001 indicated an extremely significant difference. As shown in panels (a), (b), and (c) of FIG. 6, the viral copy numbers in the lungs of mice in both the prophylactic groups and treatment groups were significantly lower than those in the PBS control group. Mice in the prophylactic groups and treatment groups exhibited fewer hyperemic and hemorrhagic lesions in the lungs. H&E staining further revealed reduced inflammatory cell infiltration, alveolar wall thickening, and alveolar hemorrhage in these groups. These findings demonstrate that the AL5E protein can effectively prevent and protect mice from NL63 coronavirus infection and significantly reduce pulmonary inflammation.

## Example 7: AL5E protein inactivates human coronavirus NL63 to protect K18-ACE2 transgenic mice from infection

To determine the effect of the AL5E protein in inactivating human coronavirus NL63 (provided by National Institute for Viral Disease Control and Prevention, Chinese Center for Disease Control and Prevention) and protecting K18-ACE2 transgenic mice from infection, 7000 PFU of coronavirus NL63 was mixed with an equal volume of the AL5E protein (prepared in Example 1) (15 mg/kg), and then the mice were infected intranasally. A nucleoside analog inhibitor, Remdesivir, without viral inactivation effect but viral inhibition effect (purchased from MCE, Cat. No. 1809249-37-3) (25 mg/kg) was used as a negative control for viral inactivation effect, and an equal volume of PBS was used as a solvent control. On day 5 post infection, the mice were euthanized and treated according to the method described in Example 6.

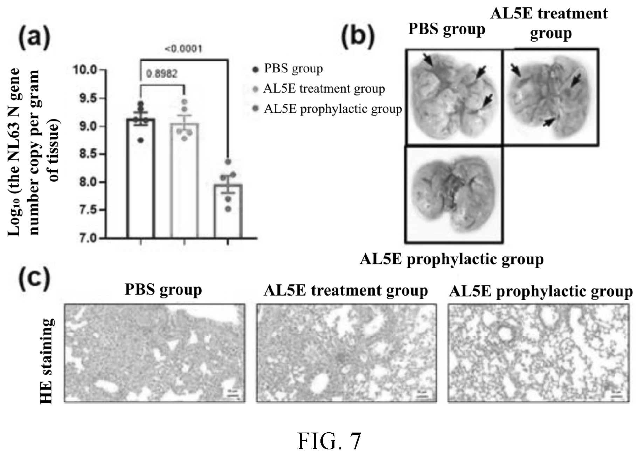

The results showed that, as shown in panels (a), (b) and (c) of FIG. 7, the virus copy number in the lungs of mice in the AL5E protein inactivation group was significantly lower than that in the PBS control group and Remdesivir treatment group, while there was no difference between Remdesivir treatment group and PBS control group. Moreover, there were decreased hyperemia and hemorrhage in the lungs of mice in the AL5E protein inactivation group. Further observation of tissue sections revealed that the lungs of mice in the AL5E protein inactivation group had decreased inflammatory cell infiltration, alveolar wall thickening, alveolar hemorrhage, and pulmonary cell necrosis and disintegration.

The above results further highlight the advantage of using a virus-inactivating protein over a viral inhibitor during the early stages of infection. Specifically, the protein exerts its effect while virus particles are still free and unbound to host cells, thereby providing superior protection to host tissues.

**Discussion**

In the present disclosure, the inventors first verified the purities and expected molecular identities of eukaryotically expressed ACE2-EK1 fusion proteins containing linkers of varying lengths, utilizing gel filtration chromatography and Coomassie Brilliant Blue staining. Subsequently, the inhibitory activities of the AL2E, AL5E, AL6E, and AL7E proteins against SARS-CoV-2 pseudovirus infection were evaluated using an established packaging and inhibition assay system for SARS-CoV-2. In previous work, the inventors constructed pseudoviruses for a series of SARS-CoV-2 variant strains by co-transfecting the viral S protein expression plasmid and the backbone plasmid pNL4-3 into 293T cells. Using this system, a series of SARS-CoV-2 protein-based entry inhibitors with potent activity were successfully designed and evaluated (for detailed methods, see Xing L., et al. A five-helix-based SARS-CoV-2 fusion inhibitor targeting Heptad Repeat 2 domain against SARS-CoV-2 and its variants of concern. Viruses. 2022 Mar 13;14(3):597). In the present disclosure, the same method was employed to evaluate the inhibitory activity of the novel AL2E, AL5E, AL6E, and AL7E proteins against pseudoviruses representing SARS-CoV-2 VOCs and novel Omicron subtypes. The results demonstrated that AL5E, which exhibited the highest efficacy among the fusion proteins with different linkers, showed a 27- to 600-fold increase in inhibitory activity compared to the ACE2 peptidase domain alone.

To further explore the effectiveness of the AL5E protein of the present disclosure in inhibiting the prototype SARS-CoV-2 strain, WHO-designated VOC strains, and novel Omicron subtypes, the inhibitory activity of the AL5E protein against the prototype SARS-CoV-2 strain, five VOC strains, and novel Omicron subtypes (BA.1 to BA.5, BF.7, XBB, and BQ.1) was detected. The results showed that the $IC_{50}$ values of the AL5E protein against different types of pseudoviruses ranged from 0.03 to 0.93 nM, falling within the low nanomolar range. More importantly, compared to the ACE2 protein alone, its inhibitory effect was increased by 27- to 600-fold. Although pseudoviruses containing the SARS-CoV-2 envelope protein can effectively mimic the receptor binding and membrane fusion processes of live virus infection, reliable assessment of drug inhibitory activity requires further validation using authentic live viruses. Therefore, this disclosure utilized the P3 Laboratory of Fudan University to evaluate the inhibitory activity of the AL5E protein against live BA.2.2 virus infection. The results showed that the $IC_{50}$ of the AL5E protein against the live BA.2.2 virus was less than 5 nM, which is consistent with the inhibitory activity observed in the pseudovirus assay.

The fusion proteins (particularly the AL5E protein) in the present disclosure not only demonstrate potent inhibitory activities against infection by pseudoviruses representing the prototype SARS-CoV-2 strain, VOC strains, and novel Omicron subtypes and live viruses, but more importantly, can directly act on free virus particles to render them non-infectious. This capability is not possessed by current small-molecule inhibitors targeting viral proteases or other polypeptide inhibitors. The inventors evaluated the inactivation activity of the AL5E protein against the prototype SARS-CoV-2 strain, five VOC strains, and novel Omicron subtypes (BA.1 to BA.5, BF.7, XBB, BQ.1) using an established protein-based viral inactivator efficacy testing system. In previous work, the inventors successfully designed and evaluated a series of potent protein-based HIV inactivators by co-incubating the virus with the protein-based inactivator, followed by precipitating viral particles with PEG-6000 and subsequent washing to remove residual proteins (for detailed methods, see Wang X., et al. Synergistic effect by combining a gp120-binding protein and a gp41-binding antibody to inactivate HIV-1 virions and inhibit HIV-1 infection. Molecules. 2021 Mar 31;26(7):1964). In the present disclosure, the same method was used to detect the inactivation activity of the novel AL5E protein against the prototype SARS-CoV-2 strain, five VOC strains, and novel Omicron subtypes (BA.1 to BA.5, BF.7, XBB, BQ.1). The results showed that the $EC_{50}$ values of the AL5E protein against different types of pseudoviruses ranged from 1.29 to 8.72 nM, falling within the low nanomolar range. More importantly, compared to the ACE2 protein alone, its inactivation effect was increased by 22- to 507-fold. These findings indicate that the AL5E protein can efficiently inactivate both the prototype SARS-CoV-2 strain and variant strains, with an inactivation effect significantly superior to that of the ACE2 protein alone.

Among coronaviruses that have posed or have the potential to pose a threat to humans, in addition to SARS-CoV-2, SARS-CoV, Human Coronavirus NL63, and bat-derived SARS-like coronaviruses (WIV1, Rs3367) all utilize ACE2 as an entry receptor. Therefore, in the present disclosure, the effectiveness of the AL5E protein in inhibiting and inactivating these strains was further evaluated. The results showed that the $IC_{50}$ values of the AL5E protein for inhibiting these viruses ranged from 0.69 to 5.90 nM, representing a 14- to 70-fold greater effect than that of the ACE2 protein alone. The $EC_{50}$ values of the AL5E protein for inactivating these viruses ranged from 1.08 to 18.13 nM, representing a 26- to 214-fold greater effect than that of the ACE2 protein alone.

The present disclosure further evaluated the prophylactic and protective effects of the AL5E protein in a mouse model of Human Coronavirus NL63 infection. The results showed that, compared to the PBS control group, administration of the protein (1.5 mg/kg) 6 hours before viral challenge, and subsequent administrations (15 mg/kg) at 12, 24, 48, 72, and 96 hours post-challenge, significantly reduced both the viral copy number in the lungs and pathological lung damage in mice. Additionally, the present disclosure evaluated the infectivity of the virus following a single inactivation treatment with the AL5E protein in the Human Coronavirus NL63 mouse model. The results showed that the viral copy number in the lungs of mice after an inactivation treatment with the AL5E protein was significantly lower than that in the PBS control group and the Remdesivir-treated group. Remdesivir, an RNA-dependent RNA polymerase (RdRp) inhibitor, does not exert a direct virus-inactivating effect.

In summary, the fusion proteins of the present disclosure (particularly the AL5E protein) are "broad-spectrum" novel viral infection inhibitors and inactivators capable of acting on all ACE2-dependent coronaviruses. They operate via a "dual-target" mechanism, wherein their two functional domains act on the viral RBD and HR1 domains, respectively, thereby simultaneously inhibiting viral infection and inactivating free virus particles, a "dual-function" capability. The core inventive concept centers on the fusion of the ACE2 peptidase domain with the EK1 polypeptide. This combination not only potentiates the antiviral activity of the ACE2 protein but also confers virus-inactivating properties upon the EK1 polypeptide, resulting in a highly effective viral inactivator. Notably, the underlying antiviral mechanism represents a previously unreported discovery. Furthermore, these protein-based inactivators demonstrate favorable *in vivo* safety profiles, highlighting their significant potential for therapeutic development.

## Claims

1. A fusion protein comprising an ACE2 peptidase domain directly linked or linked via a first linker to an HR1 domain-targeting, anti-coronavirus polypeptide therapeutic agent, wherein the ACE2 peptidase domain comprises (1) the amino acid sequence set forth in SEQ ID NO: 1; (2) an amino acid sequence having 80%, 85%, 90%, 95%, 98%, or 99% or more identity to the amino acid sequence set forth in SEQ ID NO: 1; or (3) an amino acid sequence in which one or more amino acid residues are mutated in the amino acid sequence set forth in SEQ ID NO: 1.

2. The fusion protein according to claim 1, wherein the anti-coronavirus polypeptide therapeutic agent comprises a polypeptide selected from the group consisting of SEQ ID NOs: 2, 12, and 6, or an amino acid sequence having 80%, 85%, 90%, 95%, 98%, or 99% or more identity to any one of SEQ ID NOs: 2, 12, and 6, or a derivative thereof; preferably, the derivative is a polyethylene glycol-, palmitoyl-, or cholesterol-modified derivative of the polypeptide; preferably, the anti-coronavirus polypeptide therapeutic agent is linked to a palmitic acid or cholesterol moiety via a second linker at the C-terminus;

   preferably, wherein the second linker is PEGylated;
   preferably, wherein the polypeptide is linked to a palmitoyl-modified lysine or a cholesterol-modified cysteine via a second linker at the C-terminus;
   preferably, wherein the second linker comprises (GSG)n or (GSGSG)n, wherein n is 1 or 2;
   preferably, wherein the second linker is -(GSG)n-DPEG4- or -(GSGSG)n-DPEG4-;
   preferably, wherein the mutation is selected from a substitution, an addition, an insertion, or a deletion;
   preferably, wherein the substitution is a conservative amino acid substitution;
   preferably, wherein the first linker is a flexible linker, preferably $(G)_{n1}$, $(SGG)_{n2}$, $(SGGG)_{n3}$, $(GGSGG)_{n4}$, $(GSSGG)_{n5}$, or $(GGGGS)_{n6}$, wherein n1 = 5-35, 10-30, or 20-25; n2 = 1-13, 2-10, or 3-8; n4, n5, or n6 = 1-10, 2-7, or 3-6, for example n4, n5, or n6 = 5;
   preferably, the ACE2 peptidase domain is located at the N-terminus of the fusion protein and the anti-coronavirus polypeptide therapeutic agent is at the C-terminus of the fusion protein;
   preferably, the polypeptide derivative is SLDQINVTFLDLEYEMKKLEEAIKKLEESYIDLKEL-(GSG)n-DPEG4-K-palmitic acid or a salt or ester thereof, or
   SLDQINVTFLDLEYEMKKLEEAIKKLEESYIDLKEL-(GSGSG)n-DPEG4-C-cholesterol or a salt or ester thereof, wherein n is 1 or 2.

3. The fusion protein according to claim 1 or 2, wherein the fusion protein further comprises an enzyme cleavage site and/or a purification tag at the C-terminus;

wherein preferably, the enzyme cleavage site is an HRV 3C enzyme cleavage site;
preferably, the purification tag is a His tag, such as an 8xHis tag;
preferably, the fusion protein comprises the amino acid sequence set forth in SEQ ID NO: 5 or SEQ ID NO: 7;
preferably, the fusion protein comprises a half-life-extending moiety, such as an IgG Fc binding-peptide, such as the amino acid sequence of SEQ ID NO: 13 or a variant thereof with a substitution, an insertion, a deletion, or an addition of one or more amino acids;
preferably, the fusion protein comprises ACE2 peptidase domain - first linker - EK1 polypeptide, or EK1 polypeptide - first linker - ACE2 peptidase domain, and an IgG Fc binding-peptide is linked directly or via a third linker to the ACE2 peptidase domain or the EK1 polypeptide;
preferably, the third linker is $(G)_{m1}$, $(SGG)_{m2}$, $(SGGG)_{m3}$, $(GGSGG)_{m4}$, $(GSSGG)_{m5}$, or $(GGGGS)_{m6}$, wherein m1 = 5-35, 10-30, or 20-25; m2 = 1-13, 2-10, or 3-8; m4, m5, or m6 = 1-10, 2-7, or 3-6, for example m4, m5, or m6 = 5.

4. A nucleic acid encoding the fusion protein according to any one of claims 1-3;

preferably, wherein the nucleic acid comprises a codon-optimized nucleotide sequence;
preferably, wherein the nucleotide sequence is a nucleotide sequence that is codon-optimized for expression in a eukaryotic cell or a prokaryotic cell, such as yeast or *E. coli;*
preferably, wherein the nucleic acid comprises the nucleotide sequence set forth in SEQ ID NO: 3 and/or 4;
preferably, wherein the nucleic acid comprises the nucleotide sequence selected from SEQ ID NOs: 8-11.

5. A vector comprising the nucleic acid according to claim 4;

preferably, wherein the vector comprises an expression control element, a nucleotide of a purification tag, and/or a nucleotide of a leader sequence operably linked to the nucleic acid;
preferably, wherein the expression control element is selected from a promoter, a terminator, or an enhancer;
preferably, wherein the purification tag is selected from a His tag, a GST tag, an MBP tag, a SUMO tag, or a NusA tag;
preferably, the vector is an expression vector, a cloning vector, a shuttle vector, or a viral vector, such as a plasmid or cosmid; for example, a pFuse-hIgG1-Fc2 series of plasmids or a pET series of plasmids.

6. A host cell comprising the nucleic acid according to claim 4 or the vector according to claim 5; wherein preferably the host cell is a eukaryotic cell or a prokaryotic cell; wherein preferably the eukaryotic cell is a yeast cell, an animal cell, and/or an insect cell, and/or the prokaryotic cell is an *E. coli* cell; for example, the host cell is *E. coli, Rhodococcus ruber, Bacillus subtilis,* or a yeast.

7. A composition comprising one or more of the fusion protein according to any one of claims 1-3, the nucleic acid according to claim 4, the vector according to claim 5, and the host cell according to claim 6;

preferably, wherein the composition is a pharmaceutical composition or a kit;
preferably, the composition comprises a pharmaceutically acceptable carrier, excipient, or diluent;
preferably, the composition is a solid, liquid, or gel composition;
preferably, the composition is an injectable composition or an orally administered composition;
preferably, the composition is in the form of a tablet, capsule, drop pill, aerosol, pill, powder, solution, suspension, emulsion, granule, liposome, transdermal agent, suppository, or lyophilized powder;
preferably, the composition further comprises another anti-coronavirus therapeutic agent, preferably the therapeutic agent is selected from Paxlovid, amubarvimab / romlusevimab, COVID-19 human immunoglobulin, convalescent plasma, glucocorticoids, and interleukin-6 inhibitors.

8. A method for producing the fusion protein of any one of claims 1-3, comprising:

(1) culturing the host cell according to claim 6 under a suitable culture condition;
(2) harvesting the host cell and/or culture medium comprising the fusion protein; and
(3) purifying the fusion protein;

wherein preferably, step (3) comprises, when the fusion protein comprises a purification tag, isolating the fusion protein by column separation using a ligand specific for the purification tag and/or removing the purification tag by enzymatic digestion using a tool enzyme;

preferably, the purification tag is a histidine tag, such as an 8xHis tag;

preferably, the column separation is nickel column separation;

preferably, when the fusion protein comprises an HRV 3C enzyme cleavage site, and the tool enzyme is an HRV 3C enzyme;

preferably, the method further comprises the step of modifying the fusion protein;

preferably, when the ACE2 peptidase domain is located at the N-terminus of the fusion protein and the anti-coronavirus polypeptide therapeutic agent is at the C-terminus of the fusion protein, PEGylating or adding a cholesterol moiety to the C-terminus of the anti-coronavirus polypeptide therapeutic agent.

9. Use of the fusion protein according to any one of claims 1-3, the nucleic acid according to claim 4, the vector according to claim 5, the host cell according to claim 6, and/or the composition according to claim 7 in the manufacture of a medicament or a kit for treating or preventing coronavirus infection or a coronavirus infection-related disease in a subject; wherein preferably, the coronavirus uses ACE2 as a cellular receptor; preferably, the coronavirus is one or more of severe acute respiratory syndrome coronavirus (SARS-CoV), Middle East respiratory syndrome coronavirus, and severe acute respiratory syndrome coronavirus 2 (SARS-CoV-2), HCoV, RsSHC014-CoV, or RsW1V1-CoV; preferably, the HCoV is selected from HCoV-229E, HCoV-NL63, HCoV-OC43, and HCoV-HKU1 strains; the SARS-CoV-2 is selected from the prototype, Alpha, Beta, Gamma, Delta, Lambda, BA.1, BA.2, BA.2.2, BA.2.9, BA.2.12.1, BA.2.75, BA.3, BA.4.6, BA.5, BF.7, XBB, or BQ.1 virus strain; preferably, the medicament is in the form of a tablet, capsule, drop pill, aerosol, pill, powder, solution, suspension, emulsion, granule, liposome, transdermal agent, suppository, or lyophilized powder; preferably, the subject is a human or mammal, such as a companion animal, zoo animal, or livestock animal.

10. A method for non-therapeutic purpose of treating coronavirus infection, comprising contacting a coronavirus-infected cell with the fusion protein according to any one of claims 1-3 and/or the composition according to claim 7; wherein preferably, the coronavirus uses ACE2 as a cellular receptor; preferably, the coronavirus is one or more of severe acute respiratory syndrome coronavirus (SARS-CoV), Middle East respiratory syndrome coronavirus, and severe acute respiratory syndrome coronavirus 2 (SARS-CoV-2), HCoV, RsSHC014-CoV, or RsW1V1-CoV; preferably, the HCoV is selected from HCoV-229E, HCoV-NL63, HCoV-OC43, and HCoV-HKU1 strains; the SARS-CoV-2 is selected from the prototype, Alpha, Beta, Gamma, Delta, Lambda, BA.1, BA.2, BA.2.2, BA.2.9, BA.2.12.1, BA.2.75, BA.3, BA.4.6, BA.5, BF.7, XBB, or BQ.1 virus strain.

11. A method of inhibiting coronavirus reproduction *in vitro*, or preventing or inhibiting coronavirus infection of a cell *in vitro*, comprising the step of contacting the fusion protein according to any one of claims 1-3 and/or the composition according to claim 7 with an article or sample containing or at risk of containing a coronavirus; wherein preferably, the coronavirus uses ACE2 as a cellular receptor; preferably, the coronavirus is one or more of severe acute respiratory syndrome coronavirus (SARS-CoV), Middle East respiratory syndrome coronavirus, and severe acute respiratory syndrome coronavirus 2 (SARS-CoV-2), HCoV, RsSHC014-CoV, or RsW1V1-CoV; preferably, the HCoV is selected from HCoV-229E, HCoV-NL63, HCoV-OC43, and HCoV-HKU1 strains; the SARS-CoV-2 is selected from the prototype, Alpha, Beta, Gamma, Delta, Lambda, BA.1, BA.2, BA.2.2, BA.2.9, BA.2.12.1, BA.2.75, BA.3, BA.4.6, BA.5, BF.7, XBB, or BQ.1 virus strain.

FIG. 1

FIG. 2

FIG. 2 (continued)

FIG. 2 (continued)

**Inhibition effect on live BA.2.2 virus**

FIG. 3

FIG. 4

FIG. 4 (continued)

FIG. 4 (continued)

FIG. 4 (continued)

(a)

FIG. 5

(b)

FIG. 5(continued)

FIG. 6

FIG. 7

## INTERNATIONAL SEARCH REPORT

| International application No. |
| --- |
| **PCT/CN2024/126450** |

| A. | CLASSIFICATION OF SUBJECT MATTER |
| --- | --- |

C07K19/00(2006.01)i; C07K14/165(2006.01)i; C12N9/48(2006.01)i

According to International Patent Classification (IPC) or to both national classification and IPC

| B. | FIELDS SEARCHED |
| --- | --- |

Minimum documentation searched (classification system followed by classification symbols)

IPC: C07K, C12N

Documentation searched other than minimum documentation to the extent that such documents are included in the fields searched

Electronic data base consulted during the international search (name of data base and, where practicable, search terms used)

WPABS, CNTXT, VEN, DWPI, WPABSC, ENTXT, CJFD, CNKI, Web of Science, Elsevier Science, PubMed, 百度学术搜索, BAIDU SCHOLAR SEARCH: 新冠病毒, 融合蛋白, ACE2, 肽酶, HR1, 抑制, EK1, 接头, 连结子, fusion protein, peptidase, inhibition, linker, CoV, SARS-CoV-2; GenBank, EMBL, 中国专利生物序列检索, China Patents Biological Sequence Search: 对SEQ ID NO: 1-12的序列检索, sequence search on SEQ ID NOs: 1-12

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CAI, Yangxing et al. "A Bivalent Protein Targeting Glycans and HR1 Domain in Spike Protein Potently Inhibited Infection of SARS-CoV-2 and Other Human Coronaviruses" *Cell & Bioscience*, Vol. 11, No. 1, 08 July 2021 (2021-07-08), pages 1-14 <br> see abstract, and page 2 | 1-11 |
| Y | CN 113444710 A (SHANGHAI IMMED THERAPEUTICS CO., LTD.) 28 September 2021 (2021-09-28) <br> see claims 1-16, and description, paragraphs 3, 6, 43, and 59 | 1-11 |
| Y | US 2023193235 A1 (UNIVERSITY OF ILLINOIS) 22 June 2023 (2023-06-22) <br> see description, paragraphs 102, 105, and 106 | 1-11 |
| Y | CN 107022008 A (FUDAN UNIVERSITY) 08 August 2017 (2017-08-08) <br> see description, paragraphs 4 and 11 | 1-11 |
| A | CN 115746148 A (NATIONAL INSTITUTE OF PATHOGEN BIOLOGY, CAMS & PUMC) 07 March 2023 (2023-03-07) <br> see claims 1-2 | 1-11 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **26 November 2024** | **11 January 2025** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/126450** |

### C.    DOCUMENTS CONSIDERED TO BE RELEVANT

| Category* | Citation of document, with indication, where appropriate, of the relevant passages | Relevant to claim No. |
| --- | --- | --- |
| A | CN 116440285 A (SHANXI JINBO BIOPHARMACEUTICAL LTD.; FUDAN UNIVERSITY) 18 July 2023 (2023-07-18)<br>see claims 1-12 | 1-11 |
| A | WO 2023075697 A2 (AGENCY FOR SCIENCE, TECHNOLOGY AND RESEARCH) 04 May 2023 (2023-05-04)<br>see claims 1-6 | 1-11 |

Form PCT/ISA/210 (second sheet) (July 2022)

## INTERNATIONAL SEARCH REPORT

International application No.

**PCT/CN2024/126450**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

    ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT
### Information on patent family members

International application No.

**PCT/CN2024/126450**

| Patent document cited in search report | | | Publication date (day/month/year) | Patent family member(s) | | | Publication date (day/month/year) |
|---|---|---|---|---|---|---|---|
| CN | 113444710 | A | 28 September 2021 | None | | | |
| US | 2023193235 | A1 | 22 June 2023 | KR | 20220154796 | A | 22 November 2022 |
| | | | | WO | 2021188576 | A1 | 23 September 2021 |
| | | | | BR | 112022018527 | A2 | 25 October 2022 |
| | | | | AU | 2021239879 | A1 | 29 September 2022 |
| | | | | EP | 4121170 | A1 | 25 January 2023 |
| | | | | CA | 3174683 | A1 | 23 September 2021 |
| | | | | JP | 2023518038 | A | 27 April 2023 |
| CN | 107022008 | A | 08 August 2017 | None | | | |
| CN | 115746148 | A | 07 March 2023 | None | | | |
| CN | 116440285 | A | 18 July 2023 | None | | | |
| WO | 2023075697 | A2 | 04 May 2023 | JP | 2024540722 | A | 01 November 2024 |
| | | | | EP | 4426825 | A2 | 11 September 2024 |
| | | | | WO | 2023075697 | A3 | 15 June 2023 |

Form PCT/ISA/210 (patent family annex) (July 2022)

## REFERENCES CITED IN THE DESCRIPTION

### Patent documents cited in the description

- CN 202311398330
- CN 107022008 A
- CN 115057914 A
- CN 202310423264
- CN 202180001804

### Non-patent literature cited in the description

- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277
- **RICE et al.** *EMBOSS: The European Molecular Biology Open Software Suite*, 2000
- **L. LU** ; **Q. LIU** ; **Y. ZHU** ; **K.-H. CHAN** ; **L. QIN** ; **Y. LI** ; **Q. WANG** ; **J. F.-W. CHAN** ; **L. DU** ; **F. YU**. Structure-based discovery of Middle East respiratory syndrome coronavirus fusion inhibitor.. *Nat. Commun.*, 2014, vol. 5, 3067
- **XING L. et al.** A five-helix-based SARS-CoV-2 fusion inhibitor targeting Heptad Repeat 2 domain against SARS-CoV-2 and its variants of concern.. *Viruses.*, 13 March 2022, vol. 14 (3), 597
- **XING L. et al.** A five-helix-based SARS-CoV-2 fusion inhibitor targeting Heptad Repeat 2 domain against SARS-CoV-2 and its variants of concern. *Viruses.*, 13 March 2022, vol. 14 (3), 597
- **WANG X. et al.** Synergistic effect by combining a gp120-binding protein and a gp41-binding antibody to inactivate HIV-1 virions and inhibit HIV-1 infection.. *Molecules.*, 31 March 2021, vol. 26 (7), 1964